(19) Europäisches Patentamt

European Patent Office

Office européen des brevets

(11) **EP 1 488 669 B1**

(12) **FASCICULE DE BREVET EUROPEEN**

(45) Date de publication et mention
de la délivrance du brevet:
**13.09.2006 Bulletin 2006/37**

(21) Numéro de dépôt: **03710073.2**

(22) Date de dépôt: **28.03.2003**

(51) Int Cl.:
***H05H 1/24*** *(2006.01)*      ***H05H 1/44*** *(2006.01)*

(86) Numéro de dépôt international:
**PCT/IB2003/001168**

(87) Numéro de publication internationale:
**WO 2003/084294 (09.10.2003 Gazette 2003/41)**

(54) **PROCEDE DE TRAITEMENT DE SURFACE PAR PLASMA ATMOSPHERIQUE ET DISPOSITIF POUR SA MISE EN OEUVRE**

VERFAHREN ZUR OBERFLÄCHENBEHANDLUNG DURCH ATMOSPHÄRISCHES PLASMA UND VORRICHTUNG ZU SEINER HERSTELLUNG

ATMOSPHERIC PLASMA SURFACE TREATMENT METHOD AND DEVICE FOR SAME

(84) Etats contractants désignés:
**AT BE BG CH CY CZ DE DK EE ES FI FR GB GR HU IE IT LI LU MC NL PT RO SE SI SK TR**

(30) Priorité: **28.03.2002 EP 02405249**

(43) Date de publication de la demande:
**22.12.2004 Bulletin 2004/52**

(73) Titulaire: **Apit Corp. S.A.**
**1950 Sion (CH)**

(72) Inventeurs:
• **KOULIK, Pavel**
**F-67113 Blaesheim (FR)**
• **SAITCHENKO, Anatolii**
**F-67400 Illkirch-Graffenstaden (FR)**
• **KRAPIVINA, Svetlana**
**F-67400 Illkirch-Graffenstaden (FR)**
• **MUSIN, Nail**
**F-67400 Illkirch-Graffenstaden (FR)**

• **CINQUALBRE, Jacques**
**F-67560 Rosheim (FR)**

(74) Mandataire: **Reuteler, Raymond Werner**
**WILLIAM BLANC & CIE,**
**Conseils en Propriété Industrielle SA,**
**25, Avenue du Pailly**
**1220 Les Avanchets/Genève (CH)**

(56) Documents cités:
**EP-A- 0 351 847      EP-A- 0 625 869**
**EP-A- 1 194 018      BE-A- 558 775**
**US-A- 3 205 338      US-A- 3 989 512**
**US-A1- 2002 030 038**

• **KOULIK P ET AL: "ATMOSPHERIC PLASMA STERILIZATION AND DEODORIZATION OF DIELECTRIC SURFACES" PLASMA CHEMISTRY AND PLASMA PROCESSING, PLENUM PRESS. NEW YORK, US, vol. 19, no. 2, juin 1999 (1999-06), pages 311-326, XP000831088 ISSN: 0272-4324**

**Description**

La présente invention concerne un procédé de traitement de surface par plasma atmosphérique de matériaux conducteurs, peu conducteurs ou diélectriques, notamment par l'activation des surfaces, et un dispositif pour la mise en oeuvre du procédé. L'invention est particulièrement bien adaptée au traitement de matériaux biologiques, tel que la cautérisation du sang dans des opérations chirurgicales.

Le traitement de surface d'un matériau peut comprendre une ou plusieurs des opérations suivantes :

-    activation superficielle notamment pour améliorer les propriétés d'adhésion ou de mouillabilité ;
-    désinfection, stérilisation ;
-    décapage ;
-    dépôt de films ;
-    cautérisation.

On connaît des procédés et dispositifs pour le traitement de surface par jets de plasma à pression atmosphérique (voir par exemple la publication : P. Koulik, Dynamical Plasma Operating (DPO) of Solid Surfaces. Plasma Jets, p. 639-653. Solonenko & Fedorchenko (Eds) VSP 1990).

Un désavantage de ces méthodes est que le plasma du jet est à de très hautes températures ($10\text{-}15.10^3$ K) puisque l'interaction du plasma avec la surface à traiter s'effectue par l'intermédiaire des particules (atomes, molécules, radicaux) activées dans le jet, ces particules étant générées par la voie thermique. Les particules activées diffusent à travers la couche limite, de manière qu'elles ne perdent pas leur énergie d'activation avant leur impact sur la surface traitée. Il en résulte des pertes importantes d'énergie, en particulier de chaleur, des effets souvent non souhaitables de surchauffe de la surface traitée, et un rendement faible du procédé. On ne peut pas traiter des tissus biologiques (plaies, brûlures, zones de sections d'organes lors d'opérations chirurgicales etc) à l'aide d'un jet de plasma qui occasionnerait des brûlures et autres dommages considérables.

Il existe des appareils pour le traitement des tissus vivants notamment la cautérisation des zones d'hémorragies lors d'opérations chirurgicales, (par exemple, l'appareil Erbotom 12C350 fabriqué en série par ERBE médical SARL Parc d'Affaires, 11 chemin de l'Industrie, 69570 Dardilly, France) à l'aide d'un arc à haute fréquence fonctionnant dans un flux d'argon entre une électrode manipulée par le chirurgien et les tissus du patient, le corps du patient étant nécessairement mis à la terre (régime unipolaire).

La cautérisation, causée uniquement par effets thermiques c'est-à-dire échauffement par effet Joule, a le désavantage qu'un volume important de tissus à travers lequel passe un courant électrique de forte densité, est endommagé lors de ce traitement. Un tel procédé ne peut pas être utilisé par le traitement de zones d'activité nerveuse intense comme le cerveau, la moelle épinière et d'autres centres nerveux déterminants.

Un autre problème rencontré est que l'arc électrique est fréquemment interrompu par des ruptures de contact et doit être chaque fois réamorcé pour continuer le traitement puisque le courant électrique passe à travers les tissus plus ou moins suintant de liquide (de sang par exemple).

Vu que la surface de contact de l'arc avec 1e tissus traité est déterminée par le diamètre très réduit des tâches électrodiques à l'interface plasma-tissus, un autre désavantage est que le traitement de larges surfaces est difficile, fastidieux et long.

L'appareil Erbotom ICC350 permet d'opérer un traitement de coagulation en régime bipolaire, l'arc électrique brûlant entre deux électrodes en direction étant soufflé vers le tissus biologique traité par un jet d'argon perpendiculaire aux lignes de courant. Un désavantage est que l'arc contourné par le jet d'argon est géométriquement instable et a tendance à s'éloigner de la zone à traiter, puisque le flux d'argon ne détermine pas la configuration de l'arc et ne force pas l'arc à contacter le relief de la surface traitée, d'où faible efficacité. Ce procédé ne permet pas de régler l'intensité de l'effet thermique de l'arc en variant la distance entre l'électrode et la surface à traiter.

Pour des raisons physiques évidentes, un tel procédé ne pourrait pas être utilisé pour traiter des corps diélectriques.

D'autre part, un tel procédé ne serait pas apte à être utilisé pour le traitement de surface métalliques, étant donné que cette surface, mise à la terre, se comporterait comme une deuxième électrode. L'action de l'arc en question sur le métal sera donc accompagnée de tous les phénomènes de dégradation typiques pour les électrodes (formation de tâches électrodiques, érosion, fusion, échauffement, destruction de la structure atomique et autres).

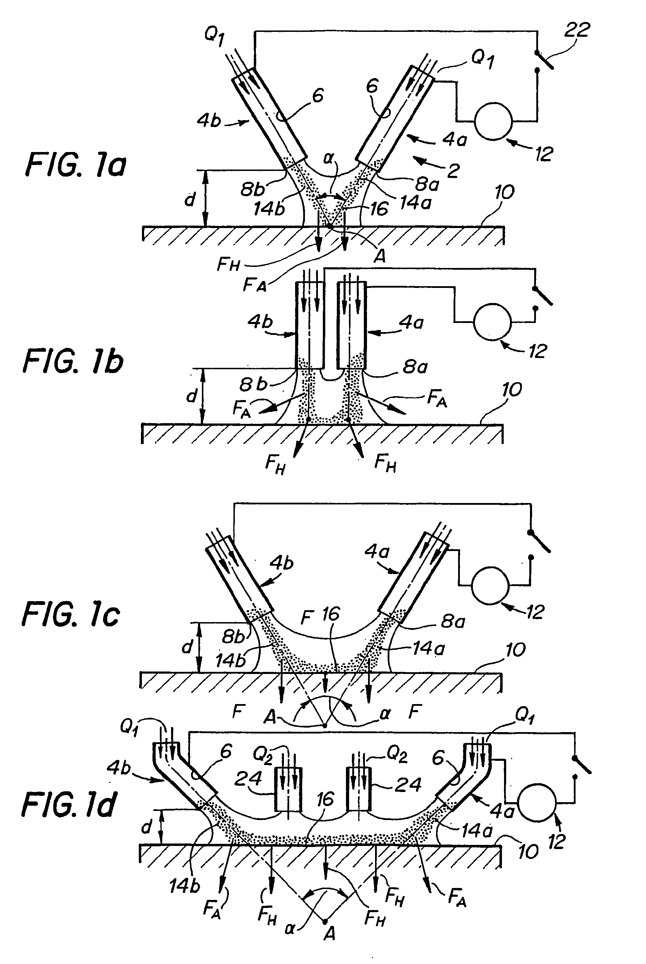

Dans la demande de brevet internationale WO 97/22369, un dispositif pour le traitement de surface par plasma, notamment pour la stérilisation, est décrit. Le dispositif de la figure 1 de cette publication comporte des électrodes métalliques filiformes disposées sous un angle donné l'un par rapport à l'autre à l'intérieur de tubes diélectriques (en quartz), à travers lesquels passe un gaz. Les tubes sont ouverts du côté de l'arc électrique généré entre les pointes des électrodes filiformes.

Les inconvénients de ce dispositif sont *inter alia :*

- La chaleur générée et le courant électrique dû aux arcs électriques sont désavantageux pour beaucoup d'applications, tels que le traitement de surface de tissus biologiques, en raison des dommages dus aux brûlures et au courant électrique passant à travers le corps à traiter. D'autre part; le procédé n'est pas économe en énergie.
- Le dispositif représente un danger de brûlure pour son utilisateur.
- Le débit de gaz est très grand vu la nécessité de refroidir le dispositif dans les conditions de faibles gradients de température.
- Les tubes en quartz se détruisent par suite du contact avec les arcs électriques issus des électrodes.
- Il est difficile d'utiliser ce dispositif dans des conditions où le matériau traité, de par sa nature, peut boucher les orifices des tubes (par exemple éclaboussures de sang).
- Le dispositif est difficilement miniaturisable.

Dans la demande de brevet européen EP 0279745, un dispositif pour le traitement de surface par plasma semblable au précédent est décrit, mais dans lequel les électrodes bipolaires filiformes sont contenues dans des porte-électrodes tubulaires métalliques jouant le rôle de conduit véhiculant un courant de fluide qui traverse la zone où est allumé un arc électrique. L'arc électrique a pour fonction d'échauffer le fluide qui le traverse avant son action sur la surface à traiter. Le courant de fluide dirige le jet de plasma, refroidit les électrodes et participe à la formation du plasma. Les désavantages du dispositif décrit dans cette publication sont, *inter alia* :

- Le traitement est uniquement un traitement thermique (par exemple le découpage d'un tissu biologique). Le résultat du traitement est une brûlure plus ou moins profonde.
- La décharge électrique est un arc influencé par les effets de pointe des électrodes des filiformes et le flux de gaz qui courbent les lignes de courant entre les extrémités des électrodes. La localisation du traitement est donc très relative et difficile à opérer.
- Le fluide utilisé, principalement l'air et l'eau, a une haute enthalpie d'ionisation dont l'action physique sur la décharge est principalement un réchauffement qui lui-même affaiblit la décharge. Le fonctionnement de l'appareil revient donc à une concurrence entre l'action d'échauffement du fluide apporté, qui refroidie la décharge et l'action de soutien de la décharge électrique qui nécessite une haute température.
- L'amorçage d'une telle décharge est subséquemment difficile, ce qui nécessite des mesures spéciales d'amorçage. Une de ces mesures consiste à rendre les électrodes creuses pour pouvoir introduire par exemple un jet d'eau ou un spray d'aérosol.
- Les pointes électrodiques sont extrêmement chaudes et représentent un danger de brûlure autant du matériau traité que de l'utilisateur.
- Le dispositif en question nécessite un réglage permanent et suivi du débit, ainsi que de la puissance électrique, ce qui complique l'opération par l'utilisateur.

Un but de l'invention est de fournir un procédé ainsi qu'un dispositif pour la mise en oeuvre du procédé, pour le traitement par plasma atmosphérique d'une surface à traiter qui sont efficaces et évitent ou minimisent les dommages aux corps à traiter, notamment des dommages dus à un échauffement excessif ou au passage du courant électrique dans le corps à traiter.

Un autre but de l'invention est de fournir un procédé ainsi qu'un dispositif pour la mise en oeuvre du procédé, pour le traitement de surface de tissus biologiques par plasma atmosphérique, qui permettent un traitement efficace et précis en évitant ou en minimisant la brûlure et l'endommagement du tissu.

Il est avantageux de fournir un procédé de traitement par plasma qui est économe en énergie.

Il est avantageux de fournir un procédé de traitement de surface par plasma atmosphérique qui est fiable et qui réduit ou évite le problème de désamorçage du plasma intempestif.

Il est avantageux de fournir un dispositif pour le traitement de surface par plasma ayant une construction simple, compacte (notamment pour le domaine médical) et autorégulatrice.

Il est avantageux de réaliser un procédé de traitement par plasma et un dispositif pour sa mise en oeuvre apte à traiter de larges surfaces de matériaux, notamment pour le traitement des brûlures.

Il est aussi avantageux de réaliser un procédé de traitement par plasma et un dispositif pour sa mise en oeuvre, apte à traiter des surfaces très réduites de matériaux, notamment pour leur utilisation en laparoscopie et en endoscopie.

Il est en outre avantageux de réaliser un procédé, et un dispositif pour sa mise en oeuvre, qui peut effectuer une opération ou une combinaison d'opérations telles que le décapage, le dépôt de film, la stérilisation, l'activation de surface, et des traitements physico-chimiques et thermiques tels que la cautérisation chirurgicale, notamment les organes très fragiles comme la rate, le foie, les reins, les organes génitaux internes, le coeur et les poumons.

Des buts de l'invention sont réalisés par un procédé selon la revendication 1, et par un dispositif selon la revendication 11 ou 12 pour la mise en oeuvre du procédé.

Premièrement, dans la présente invention, un procédé de traitement de surface par plasma atmosphérique d'objets ou matériaux conducteurs, faibles conducteurs ou diélectriques est organisé de manière à amener dans la zone de décharge un gaz ou mélange gazeux (gaz d'apport) dont l'enthalpie d'ionisation est inférieure à celle du gaz environnant (par exemple de l'air). Ceci signifie que la création d'une décharge électrique dans le gaz (ou le mélange gazeux) d'apport nécessite une quantité d'énergie inférieure à celle que nécessiterait une décharge électrique dans le gaz ambiant. Cette première condition détermine donc l'apparition et le maintien de la décharge et donc du plasma dans le flux du gaz d'apport. La configuration de ce flux dans ce cas, détermine la configuration du plasma c.à.d de l'état ionisé du gaz d'apport et donc la forme géométrique de la décharge. C'est une condition de stabilisation de la décharge électrique. Elle permet de localiser le plasma dans le flux de gaz d'apport et de le forcer à suivre la trajectoire du flux et non celle du parcours le plus court entre les électrodes légèrement modifié par des effets hydrodynamiques, en particulier de sortir des électrodes tubulaires, d'atteindre le matériau traité et d'en épouser la forme comme le fait le flux de gaz d'apport. Le plasma, cependant est alimenté par le courant électrique de la décharge et donc, tôt ou tard les électrons se fraieront un passage pour rejoindre les électrodes. Il y aura donc, à ce moment une compétition entre le flux de gaz d'apport et les forces électrodynamiques. Le résultat de cette compétition détermine la configuration finale du plasma.

Deuxièmement, le gaz d'apport a une section efficace d'interactions élastiques électrons-atomes plus petite que la section efficace d'interactions élastiques électrons-atomes du gaz environnant (de l'air par exemple). Cette deuxième condition signifie que les électrons du plasma (il suffit que le plasma soit faiblement ionisé pour que la décharge ait lieu) ont une longueur de parcours moyen par rapport aux atomes assez grande pour qu'ils accumulent une énergie cinétique importante dans le champ électrique de la décharge. Cette énergie peut atteindre l'énergie d'activation des atomes du gaz d'apport ou de particules introduites dans le plasma en aval des électrodes et donc activer chimiquement ces particules, mettant le plasma hors d'équilibre thermodynamique. A partir de ce moment, toute interaction des particules de plasma avec celles des matériaux traités, notamment leur activation, devient une interaction appelée plasmochimique et est beaucoup plus efficace et rapide que les interactions principalement chimiques ou thermiques habituelles, correspondant à l'art antérieur.

Troisièmement, une condition nécessaire pour que le plasma puisse atteindre l'état d'excitation non thermique est:

$$E > J\, n\, Q\, /\, e$$

où : E est l'intensité du champ électrique créant la décharge électrique
J est l'énergie d'activation des particules du gaz
n est la densité des particules du gaz
Q est la section efficace des collisions élastiques des électrons avec les particules du gaz
e est la charge de l'électron

Selon l'invention, l'intensité du champ électrique E est réglée de manière à satisfaire la condition suivante:

$$(J\, n\, Q\, /\, e)\ \text{gaz d'apport} < E < (J\, n\, Q\, /\, e)\ \text{gaz ambiant.}$$

Le comportement de la décharge sera également déterminé par les débits de plasma dans les deux colonnes. Il y a deux débits limites. Le premier, $G_1$, est un débit faible, où la décharge a la forme d'un arc entre les deux tubes d'apport de gaz. Ce régime de débit peut être facilement visualisé puisque, en fait, entre les deux électrodes (tubes) se crée un arc. La température y atteint ~ 6000-7000°C. Ce régime correspond à l'état de la technique.

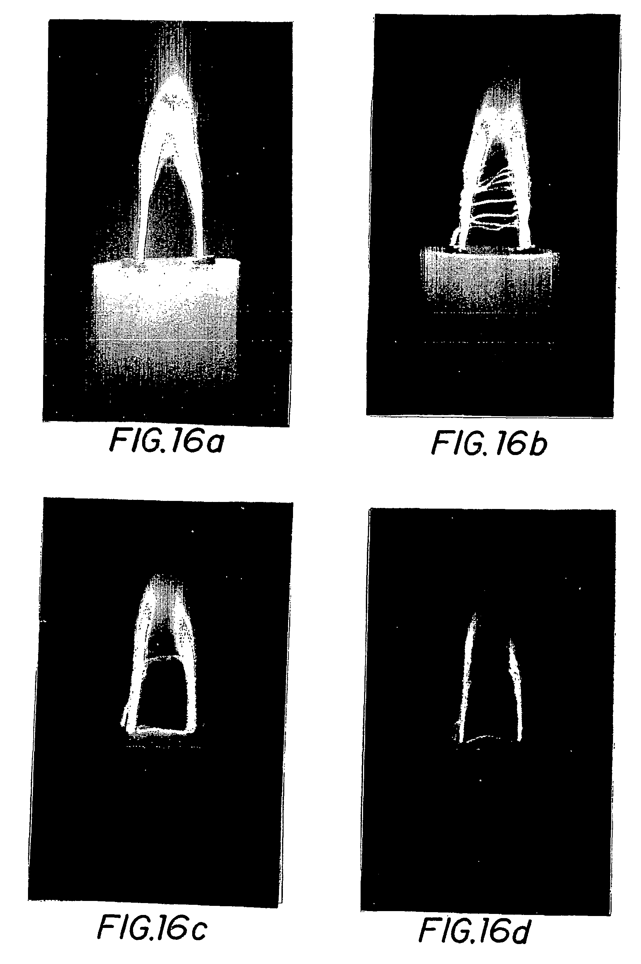

Quand on augmente le débit de gaz alimentant la décharge, l'arc s'étire, puis disparaît. Il reste deux colonnes ou jets entre lesquelles on a une décharge non-autonome. Elle est non autonome car elle ne peut pas exister sans l'alimentation de la zone entre les colonnes par des ions et électrons (plasma) venant de ces colonnes. Ces deux colonnes de plasma jouent le rôle d'électrodes. Mais ce sont des électrodes spécifiques : une électrode métallique, sous forme de pointe par exemple, n'émet que des électrons. L'électrode sous forme de colonne de plasma émet des électrons mais aussi des ions (du plasma) par convection et par photo-ionisation. Cette électrode de plasma est donc très spécifique et se diffère d'une électrode métallique telle que connue dans l'état de la technique. L'effet principal exploité dans la présente invention est l'effet de convection, dû au débit de plasma dans les électrodes de plasma (jets, colonnes de plasma). Plus grand est le débit de gaz dans les jets de plasma, plus grande doit être la tension entre les électrodes de plasma pour soutenir la décharge. A un certain débit apparaissent entre les électrodes des décharges de claquage (voir fig. 16b en particulier - on voit les filaments de claquage).

Il existe un débit maximum $G_2$, pour lequel les colonnes de plasma ne sont plus capables de créer, par convection, un milieu ionisé (plasma) assez conducteur pour soutenir la décharge. Le courant s'arrête, la décharge disparaît (les électrodes sous forme de jets de plasma aussi). Ce débit limite est évidemment détectable. Donc, le débit G de gaz (égal le plus souvent dans les deux électrodes de plasma) qui permet d'établir le régime de décharge revendiqué doit nécessairement se trouver entre deux limites :

$$G_1 \leq G \leq G_2$$

On peut exploiter mieux cette situation en envoyant entre les électrodes de plasma un gaz supplémentaire par une ou des tuyères (ou tubes) dont l'axe est dans le plan des axes des électrodes de plasma symétriquement par rapport à ces électrodes. Dans ce cas, si on appelle G* le débit de gaz supplémentaire, on aura, de nouveau, pour que se réalisent les conditions de la présente invention, l'inégalité :

$$G_1^* \leq G^* \leq G_2^*$$

Ou $G_1^*$ est la valeur de G* en-dessous de laquelle on a un arc classique entre les tubes-électrodes et $G_2^*$ est le débit maximum au-dessus duquel la décharge est soufflée, c.à.d arrête d'exister.

Le gaz supplémentaire, peut être d'une nature chimique différente du gaz d'apport des électrodes et du gaz ambiant (par exemple, $CO_2$, $N_2$, $O_2$, $NH_3$, vapeurs organométalliques et autres, ou leurs mélanges).

La décharge non-autonome permet de fournir sur la surface à traiter, des particules (atomes, molécules, radicaux) fortement activées, qui conditionnent les traitements de surface tels que la cautérisation de plaies, la désinfection, l'activation de la surface avant dépôt de film, de teinture, le décapage de la surface, le dépôt de film, et la création d'alliages superficiels.

L'expérience a montré que ce procédé et le dispositif correspondant permettent d'exécuter des traitements de surface à l'aide du jet de particules activées issu du dispositif de l'invention, à basse température (T~30-40°C) de la surface à traiter. On peut donc traiter des matériaux thermofuges. Le traitement est dû à la réaction plasmochimique sur la surface et non à un effet thermique à l'endroit de contact de l'arc avec la surface comme connu de l'état de la technique. Le plasma créé entre les électrodes est nettement un plasma hors d'équilibre thermodynamique dans lequel la concentration des particules activées (atomes, molécules, radicaux) est particulièrement élevée, sans pour autant que la température du plasma soit élevée, et ce, notamment, à pression atmosphérique.

En résumé, par les mesures décrites ci-dessus, l'on évite ou réduit fortement la formation de plasma thermique, tel que généré par les arcs électriques autonomes, et l'on optimise la formation d'atomes ou de molécules activés dans la zone de traitement. Ceci permet d'effectuer des traitements plasmo-chimiques sur la surface à traiter (par exemple activation de surface, décapage plasmo-chimique, dépôt de film hors d'équilibre chimique, et autres), avec un plasma à basse température, économe en énergie et à relativement faible courant électrique, mais très riche en atomes, molécules et radicaux activés pour optimiser les réactions plasmo-chimiques voulues.

Le dispositif peut comprendre deux ou plus de deux électrodes sous forme de tubes métalliques de petit diamètre par lesquels est introduit le gaz d'apport dans la zone de la décharge. Les électrodes sont dirigées l'une vers l'autre sous un angle entre 0 et 180° et sont insérées dans un corps isolant qui les fixe l'une par rapport à l'autre.

Le corps isolant des électrodes tubulaires peut comporter une tuyère supplémentaire par laquelle passe le gaz d'apport dont la composition est déterminée par les exigences du traitement. Le dispositif peut être inséré dans un corps en métal ou en plastique à l'intérieur duquel pénètrent les fils électriques et les tuyaux pour l'alimentation des gaz. L'extrémité fonctionnant du dispositif peut être protégée par un étui en plastique ou en céramique. Les électrodes sont connectées à une source de courant soit continu, soit alternatif, soit à haute fréquence, soit triphasé ou à une source d'impulsions électriques.

Le courant électrique passant d'une électrode à l'autre suit d'abord le canal gazeux formé par les gaz sortant des électrodes tubulaires, gaz dont le potentiel et l'énergie d'ionisation sont inférieurs respectivement au potentiel et à l'énergie (enthalpie) d'ionisation du gaz ambiant (typiquement de l'air). Ensuite, il se distribue entre les deux jets de plasma formés (voir figures 16a à 16d). Au fur et à mesure d'augmentation de la différence de potentiel électrique entre les électrodes, peuvent apparaître des canaux de claquage entre les deux jets de plasma qui jouent le rôle d'électrodes.

Il est possible, suivant les exigences du traitement, d'arranger la configuration des électrodes de manière à obtenir une zone plus ou moins large balayée par le flux de plasma comprimé par le gaz stabilisant contre la surface du matériau traité. Dans une forme d'exécution du procédé on peut, par exemple, effectuer des traitements très locaux, lorsque la zone à traiter se trouve à proximité du point d'intersection A des axes des deux électrodes (voir fig. 1a).

Dans une autre forme d'exécution, on peut exécuter des traitements très locaux par des orifices très restreints comme en coelioscopie ou en endoscopie, quand les électrodes tubulaires sont parallèles l'une à l'autre, le gaz stabilisant et les forces d'Ampère empêchant le court-circuit du courant entre les électrodes et le forçant de se propager jusqu'au contact avec le matériau à traiter (voir fig. 1b).

Dans une autre forme d'exécution, on peut au contraire effectuer des traitements sur une large zone en la balayant par la colonne d'arc stabilisé, comprimée à la fois par les forces hydrodynamiques ($F_H$) et les forces d'Ampère ($F_A$), contre la surface à traiter, les électrodes tubulaires, dans ce cas, étant fortement écartées l'une de l'autre et le point d'intersection A de leurs axes se trouvant géométriquement en-dessous de la surface à traiter (voir fig.1c).

Dans une autre forme d'exécution, pour élargir encore plus la zone traitée, on peut écarter davantage (par rapport au cas précédent) les électrodes tubulaires tout en ajoutant des tubes intermédiaires dans le plan des électrodes tubulaires et essentiellement tubulaires à la surface à traiter des jets de gaz supplémentaires alimentés par ces tubes intermédiaires, le potentiel et l'énergie (enthalpie) d'ionisation du gaz supplémentaire étant inférieurs à ceux du gaz ambiant, de manière à maintenir par des forces hydrodynamiques le canal de plasma en contact avec la surface à traiter à grande distance des électrodes, là ou les forces d'Ampère sont insuffisantes à cet effet. Au lieu de tubes intermédiaires, on peut fournir un tube aplati dans le plan des électrodes de manière à comprimer uniformément le plasma le long de la surface à traiter.

Le procédé selon l'invention permet de traiter les matériaux diélectriques. Le courant électrique ne pénètre pratiquement pas à l'intérieur des matériaux faiblement conducteurs, tels que les tissus biologiques par exemple, lors d'opérations chirurgicales. Le traitement de matériaux métalliques est possible également. Dans ce cas une partie du courant passe superficiellement à travers le métal. L'expérience montre qu'en réalité, pour un métal refroidi, cette quantité de courant est faible vu que la surface métallique est recouverte d'une couche mince de gaz froid, non conducteur, qui empêche le court circuit à travers le métal.

La configuration de plasma sous forme de jets convergents permet d'introduire les gaz de support dans le plasma de manière efficace, en utilisant les zones de couches limites entourant le plasma et créant des flux hydrodynamiques, la plupart du temps de caractère turbulent. Le procédé proposé permet une régulation facile, que ce soit par l'intermédiaire de la source de courant, par celle des débits des gaz porteurs et/ou par la variation des dimensions géométriques et de la distance des électrodes au matériau traité.

Dans une forme d'exécution pour le traitement de surface étendues, par exemple de brûlures de tissus biologiques, il est possible d'utiliser plusieurs ensembles d'électrodes disposées en forme de peigne de manière à recouvrir lors d'un mouvement de balayage, simultanément une grande surface du corps traité.

D'autres buts et caractéristiques avantageuses de l'invention ressortiront des revendications, de la description des formes d'exécution de l'invention ci-après et des dessins annexés dans lesquels :

Les figures 1a à 1d sont des vues en section, simplifiées, illustrant la configuration des électrodes tubulaires selon différentes formes d'exécution de l'invention;

La fig. 2 est une vue en section, simplifiée, illustrant des électrodes tubulaires dans un filtre hydrodynamique pour une laminarisation du flux gazeux selon une forme d'exécution de l'invention;

La fig. 3a est une vue en section d'une partie d'un dispositif de traitement de surface selon une forme de l'invention, comprenant deux électrodes tubulaires;

La fig. 3b est une vue en section détaillée d'une partie du dispositif de la fig. 3a;

La fig. 4 est une vue en perspective d'une partie d'un dispositif selon une autre forme d'exécution avec trois électrodes tubulaires pour réaliser une décharge à trois jets, et avec un tube intermédiaire central pour l'alimentation en gaz supplémentaire;

La fig. 5 est une vue en section d'un dispositif selon une autre forme d'exécution sous forme de peigne pour le traitement de grandes surfaces;

La fig. 6 est une vue en perspective d'une partie d'un dispositif, illustrant la distribution des lignes de courant dans le cas d'une décharge aplatie par l'action d'un champ magnétique additif;

Les figures 7a à 7e représentent les schémas de différentes variantes de l'alimentation en courant de dispositifs de traitement de surface par plasma atmosphérique selon l'invention;

La fig. 8 est une vue en perspective d'un dispositif selon une autre forme d'exécution;

La fig. 9 est une vue en perspective d'une partie d'un dispositif selon l'invention illustrant le cas de l'alimentation du dispositif en impulsions de courant à haute fréquence quand les paramètres de la décharge sont réglables;

La fig. 10 est une vue en section d'un dispositif selon une autre forme d'exécution où les électrodes tubulaires sont munies de bagues à haute conductivité thermique;

Les figures 11a et 11b sont des vues en section en détail, simplifiées, de parties de paroi d'électrodes avec, respectivement sans, bague;

La fig. 12 est une vue en section d'un dispositif selon une autre forme d'exécution où les électrodes tubulaires ont un canal d'écoulement sous forme de tuyère de Laval;

La fig. 13 est une vue en section d'un dispositif selon une autre forme d'exécution, adapté notamment pour la coagulation du sang et la stérilisation avec une électrode intérieure et une électrode extérieure en forme de cône, les deux électrodes étant coaxiales;

La fig. 14 est une vue en section d'un dispositif selon une autre forme d'exécution avec une électrode monolithe et une électrode tubulaire;

La fig. 15 est une photographie d'un dispositif selon l'invention montrant la formation d'un plasma;

Les figures 16a à 16d sont des photographies d'un dispositif selon l'invention illustrant différentes possibilités d'organisation de la décharge entre électrodes tubulaires, en particulier la formation d'un plasma non-autonome entre deux jets de plasma, jouant le rôle d'électrode de plasma;

Les figures 17a à 17f sont des graphiques représentant, respectivement, les résultats des Tableaux 1 à 6 des exemples 1 à 6 ci-après, et les figures 17g et 17h sont des graphiques représentant les résultats du Tableau 8 de l'Exemple 7 ci-après.

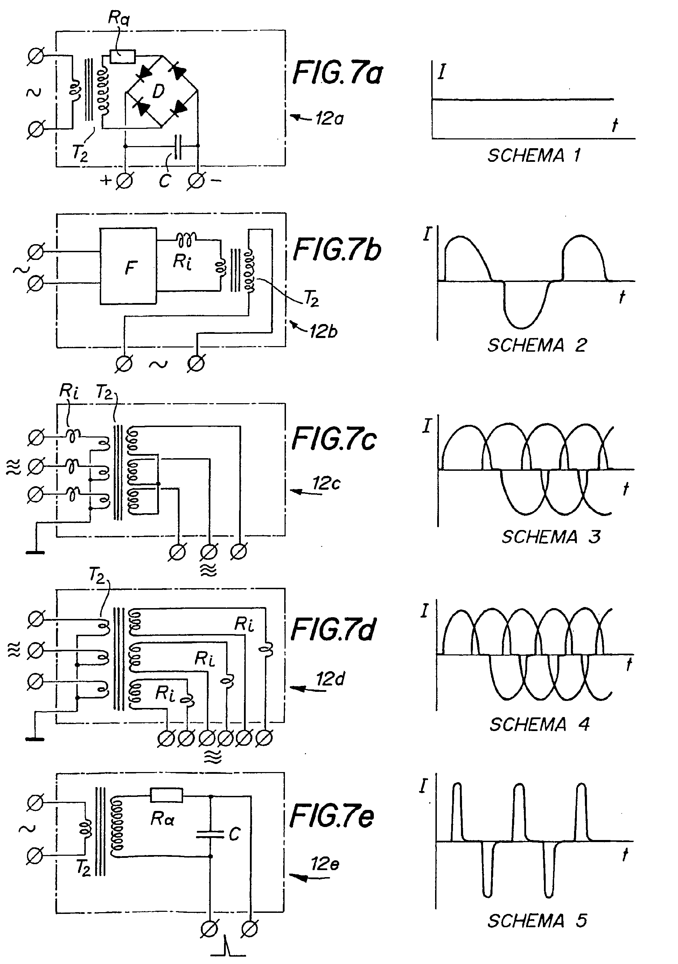

Les figures 1a à 1d montrent, de manière simplifiée, des éléments principaux d'un dispositif de traitement de surface par plasma atmosphérique selon l'invention. Le dispositif comprend un système d'électrodes tubulaires 2 comprenant au moins deux électrodes tubulaires 4a, 4b, alimentées par un gaz d'apport $Q_1$ s'écoulant dans des canaux centraux 6 des électrodes. Dans les formes d'exécution des figures 1a, 1c, 1d, les axes $A_1$, $A_2$ des électrodes sont disposées l'une par rapport à l'autre sous un angle $\alpha$ et se recoupent au point A. Les extrémités 8a, 8b des électrodes sont disposées à une distance d d'une surface à traiter 10, de relief quelconque. Les électrodes tubulaires 4a, 4b sont connectées à un circuit d'alimentation de courant 12, qui peut avoir un schéma électrique tel qu'illustré dans l'une des figures 7a à 7e.

La décharge électrique génère, à partir de chaque électrode tubulaire, un jet de plasma 14a, 14b, qui agit comme continuation de l'électrode tubulaire, c'est-à-dire que les jets agissent en tant qu'électrodes. Les paramètres de l'énergie électrique fournie aux électrodes sont réglés en fonction des paramètres du flux de gaz d'apport $Q_1$ (propriétés, débit) afin d'obtenir, dans une zone de traitement 16, un plasma non-autonome formé principalement d'un gaz activé. Le choix du gaz d'apport et le contrôle des paramètres du débit de gaz d'apport et de l'intensité du champ électrique, compte tenu des caractéristiques du gaz ambiant qui est dans le cas général de l'air (cela peut être, en coloscopie, du $CO_2$), permettent donc d'éviter ou de réduire fortement la création d'un plasma thermique dans la zone de traitement entre les électrodes et d'optimiser l'utilisation de l'énergie à la création de gaz activé pour les réactions plasmo-chimiques sur la surface à traiter. Ceci permet également de réduire le courant électrique, réduisant donc les problèmes liés au passage d'un courant électrique dans l'objet à traiter. Le gaz d'apport a une enthalpie d'ionisation inférieure à celle du milieu gazeux ambiant qui serait dans le cas général de l'air. Le gaz d'apport peut être un gaz rare (tel que de l'argon) ou un mélange gazeux (e.g. comprenant $NH_3$, O2, $N_2$, $CO_2$, vapeurs organométalliques, fréons, etc. et leurs mélanges) dépendant des réactions plasmo-chimiques souhaitées et le type de traitement à effectuer (stérilisation, dépôt, etc.).

La décharge de plasma à pression atmosphérique créée entre les extrémités des électrodes est mise en contact avec la surface à traiter sous l'action des forces hydrodynamiques $F_H$ et des forces d'Ampère résultant du passage du courant dans les zones coaxiales aux électrodes et du champ magnétique que ce courant créé, dans une direction perpendiculaire à la direction du courant dans la zone de contact 20 de la colonne de plasma avec la surface à traiter.

La source de courant 12 équipée d'un commutateur 22 peut être une source de courant continu, alternatif, unipolaire ou bipolaire, en impulsions, à haute fréquence, à basse ou haute tension, selon les applications.

La fig. 1a illustre le cas où un traitement local est effectué en réglant la distance d séparant les extrémités des électrodes de la surface à traiter de manière que le point d'intersection A des axes $A_1$, $A_2$ des électrodes tubulaires se trouve sur la surface à traiter 10. Ce traitement correspond à une concentration d'énergie maximale sur une zone de traitement de surface minimale, c.à.d à une densité d'énergie maximale.

Le dispositif selon la fig. 1b, permet d'obtenir une densité d'énergie, localement légèrement inférieure au cas de la fig. 1a. En effet les électrodes tubulaires sont disposées parallèlement l'une à l'autre. Le comportement des flux de plasma issus des électrodes est dû principalement à la concurrence entre les forces d'Ampère $F_A$ entre les deux colonnes de plasma dans lesquelles les courants électriques sont antiparallèles, ces forces repoussant les deux jets de plasma l'un de l'autre, et les forces hydrodynamiques $F_H$, résistant à cette répulsion. La jonction des lignes de courant s'effectue le long de la surface à traiter. L'avantage de cette variante est que les électrodes tubulaires 4a, 4b peuvent être de très petit diamètre. On peut donc l'utiliser pour des applications médicales, telles que la coagulation du sang en coelioscopie et en endoscopie.

L'arrangement des électrodes selon la fig. 1c correspond au cas où on a besoin d'une large zone de traitement. Les électrodes sont espacées l'une de l'autre, le point d'intersection de leurs axes A étant en-dessous de la surface à traiter. La zone de plasma de traitement 16 est compressée contre la surface à traiter 10 grâce au concours des forces hydrodynamiques $F_H$ et des forces d'Ampère $F_A$. Cet arrangement peut être utilisé pour le traitement de brûlures, par exemple.

Quand il est nécessaire de traiter des zones encore plus étendues, on peut utiliser la configuration illustrée à la fig.1d. Elle diffère du cas de la fig.1c en ce que, pour faciliter le passage du courant le long de la surface à traiter 10, et pour comprimer le plasma 16 contre la surface à traiter 10 sur l'étendue de la zone à traiter, on ajoute des flux de gaz de support $Q_2$ soit à l'aide de tubes ou canaux d'alimentation de support 24, par exemple perpendiculaires à la surface, soit par une tuyère de support aplatie, soufflant un flux de gaz de support $Q_2$ sous forme de rideau essentiellement perpendiculaire à la surface à traiter 10. Le gaz de support $Q_2$ peut être identique ou différent du gaz d'apport $Q_1$, selon les traitements à effectuer.

Dans les cas des figures 1c et 1d, le traitement d'une surface étendue est obtenu par un balayage le long de la surface à traiter dans la direction perpendiculaire à la direction du courant I engendrant le plasma le long de la surface.

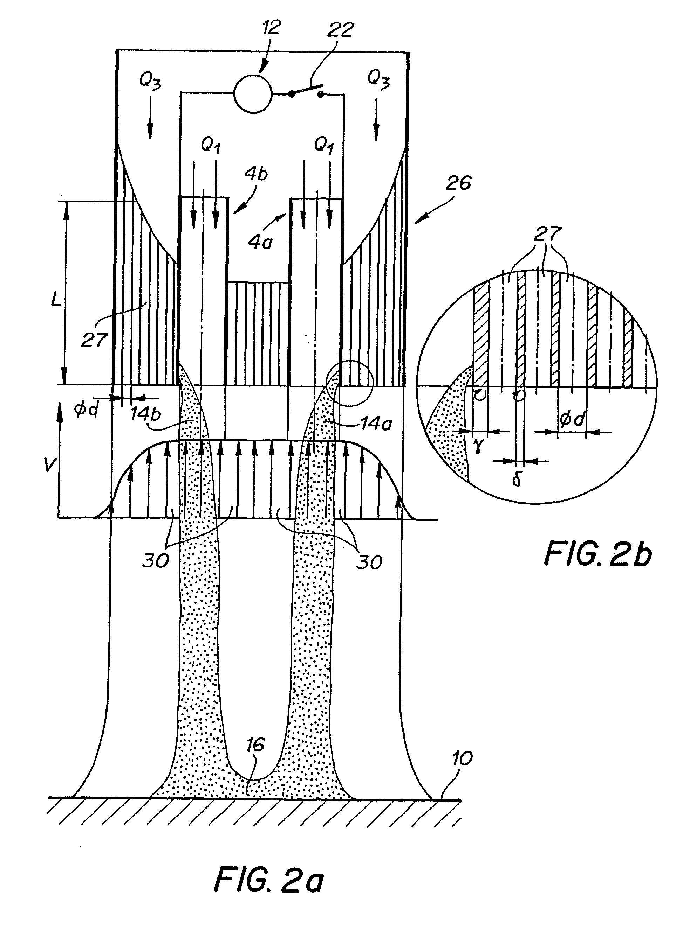

Un autre mode de réalisation du procédé revendiqué consiste à insérer les électrodes tubulaires dans un dispositif laminarisant, par exemple, un filtre hydrodynamique 26 avec une multitude de petits canaux longitudinaux 27, sous forme de nid d'abeilles (honeycomb), de manière à enrober la zone de formation du flux de plasma dans un flux laminarisant de gaz (tel que montré dans les figures 2a et 2b). Pour ce les conditions pour les dimensions caractéristiques du dispositif sont les suivantes :

$$\mathrm{Re}_{local} = \rho_{lam} v\delta/\mu_{lam} \sim \rho_{lam} v\gamma/\,\mu_{lam} \sim \rho_{sup} v\gamma/\,\mu_{sup} < 10^3$$

où $\rho$, $v$ sont respectivement la densité et la vitesse des gaz, $\delta$ et $\gamma$ les dimensions caractéristiques (voir figures 2a et 2b) et $\mu$ la viscosité des gaz et Re étant le nombre de Reynolds. Les indexes lam et sup rapportent ces paramètres aux gaz laminarisant et au gaz de support, respectivement. Dans cette forme d'exécution, les instabilités (turbulences) locales sont absorbées par le milieu et ne se développent pas laissant le flux laminaire.

Le filtre hydrodynamique 26 peut correspondre à une pluralité de tubes 27 de faible diamètre d et dont l'épaisseur des parois $\delta$ est très faible ($\delta \ll d$), la longueur L de ces tubes variant de manière à minimiser ou annuler le gradient de vitesses v du gaz dans la zone d'interface 30 entre les électrodes tubulaires et l'air ambiant. Un gaz supplémentaire $Q_3$ différent du gaz d'apport $Q_1$ alimentant les électrodes 4a, 4b (argon par exemple) peut être utilisé à cet effet.

Les avantages de cette variante sont, *inter alia,* que :

- le flux de plasma étant laminarisé même à des nombres de Reynolds supérieurs à $10^3$, les échanges (thermiques, hydrodynamiques, et de composition) sont de caractère moléculaire et non convectif et donc de beaucoup plus petite amplitude. Les pertes correspondantes sont fortement réduites ;
- la laminarisation du flux permet de porter le plasma sans pertes d'énergie d'activation à plus grande distance. En d'autres termes, la longueur du jet de plasma peut être de beaucoup supérieure à celle du jet de plasma sans dispositif laminarisant ;
- le flux de gaz laminarisant enrobant le plasma peut, en même temps le protéger de toute contamination venant soit de l'air ambiant, de l'oxygène de l'air, des poussières, de divers microorganismes. Un dispositif de cette sorte peut donc être utilisé non seulement comme cautériseur de plaies sanglantes, de sections chirurgicales et de brûlures mais aussi comme stérilisateur à action locale ou à action superficielle étendue.

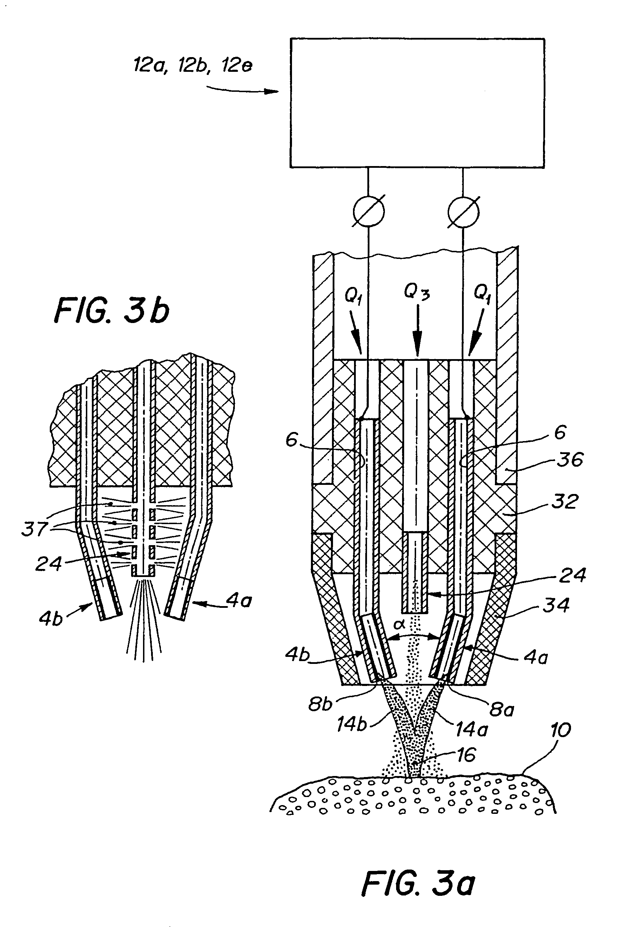

Un autre mode de réalisation du procédé revendiqué consiste à connecter les électrodes tubulaires à une source de courant à haute fréquence et d'engendrer le plasma à l'intérieur de tubes en matériau diélectrique coaxiaux aux électrodes, comme montré dans les figures 3a et 3b. L'avantage de ce mode de réalisation est que le plasma est généré uniformément à l'intérieur des tubes diélectriques. Il n'est pas contaminé par le matériau des électrodes suite à un échauffement local dans des zones de contact de la décharge avec les électrodes comme c'est le cas quand on utilise une alimentation unipolaire. En plus, le plasma est hautement hors d'équilibre thermodynamique ce qui permet d'exécuter des traitements d'activation de surface.

La fig. 3 montre un dispositif de traitement de surface par plasma comprenant deux électrodes tubulaires 4a, 4b montées dans un corps diélectrique 32. Les extrémités 8a, 8b des électrodes sont recourbées de manière à former un angle $\alpha$ entre elles, l'angle $\alpha$ pouvant varier entre 0° et 180°, mais étant avantageusement entre 20° et 40°, de préférence environ 30°. Les électrodes sont exécutées sous forme de fins tubes, par exemple de diamètre 1-3mm, à parois minces, de l'ordre d'un dixième du diamètre des tubes, en métal de haute conductivité thermique et stabilité chimique (par exemple, cuivre, argent, laiton). Un gaz d'apport $Q_1$ de faible énergie d'ionisation (par exemple du xénon) est introduit dans les canaux centraux 6 des électrodes tubulaires. La décharge électrique alimentée par le circuit d'alimentation de courant 12 est engendrée dans le flux de gaz d'apport $Q_1$ et forme deux jets de plasma 14, 14b à partir des électrodes tubulaires. Un gaz supplémentaire $Q_3$, différent du gaz d'apport, peut être introduit dans la zone de décharge par une tuyère 24 située entre les électrodes 4a, 4b. Ce gaz supplémentaire est éventuellement de l'oxygène, de l'azote, de l'oxyde carbonique ou autres, ou leur mélanges. Le choix de la composition de ce gaz et de son débit s'effectue en fonction des exigences de l'application. La tuyère centrale peut avoir des perforations radiales 37 pour assurer que le flux de gaz supplémentaire entre les électrodes se fait radialement et dans la direction des jets de plasma.

Une paroi de protection 34, entourant les électrodes et s'étendant au delà de l'extrémité 8a, 8b des électrodes, empêche toute brûlure accidentelle au contact de la surface ou du corps à traiter avec les électrodes tubulaires échauffées.

Les manipulations du dispositif s'effectuent en déplaçant le corps 36 du dispositif dans lequel sont introduits, par des tubes flexibles, les gaz et l'électricité (non montré sur la figure).

Les électrodes exécutées sous forme de tubes de faible diamètre par lesquels le gaz d'apport passe, apportent, *inter alia,* les avantages suivants :

- On peut rapprocher les électrodes au maximum et donc miniaturiser le dispositif
- Grâce à la petite surface de section des électrodes tubulaires, le flux de chaleur vers le corps diélectrique est faible. Les pièces du dispositif restent froides pendant son fonctionnement, même en régime stationnaire.
- Le flux de gaz d'apport est suffisant pour refroidir l'extrémité des électrodes. Un refroidissement à l'eau n'est pas nécessaire. Le dispositif fonctionne dans un régime d'auto-refroidissement ce qui en détermine le haut rendement énergétique.
- L'expérience a montré que le dispositif fonctionne de manière fiable à faibles débits de gaz et a une ressource d'exploitation pratiquement illimitée.

Le débit de gaz par la tuyère 24 entre les jets de plasma 14a, 14b qui se rencontrent sous un angle aigu $\alpha$ assure une aspiration naturelle de gaz dans la zone de traitement 16 et son échauffement efficace $\mu$, l'activation, la dissociation et l'ionisation de ses particules.

Un large spectre de traitements est possible suivant la composition du plasma et les différents degrés d'échauffement du plasma et d'activation. La régulation des régimes de fonctionnement et de traitement se fait par le débit de gaz, la catégorie de la décharge, le courant et la tension, ainsi que la géométrie de disposition. Il est possible d'effectuer, éventuellement, un refroidissement complémentaire des électrodes par le gaz d'apport.

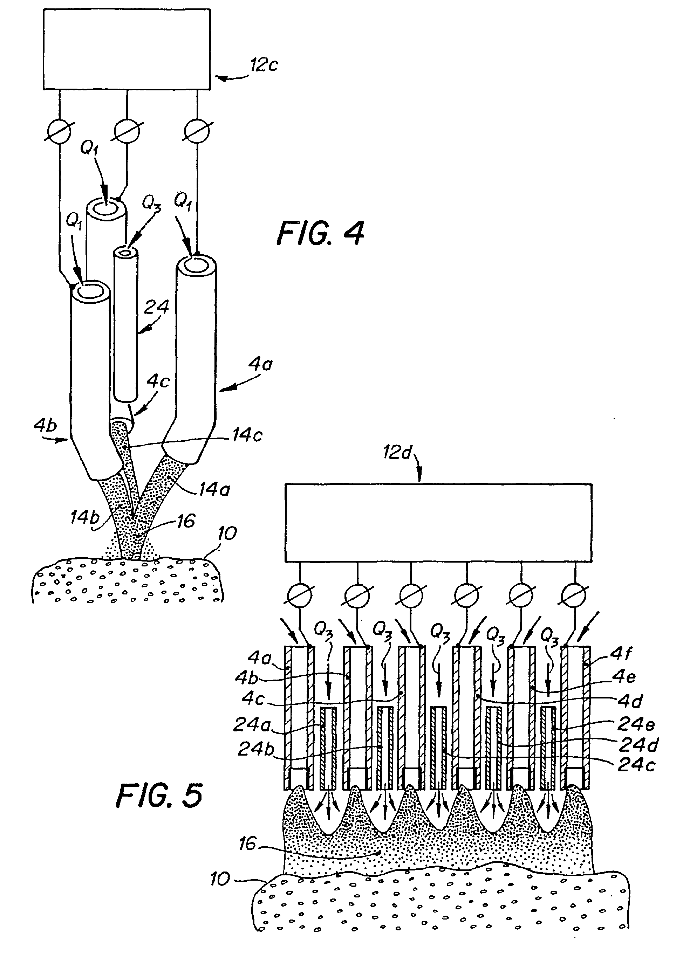

La fig. 4 illustre un dispositif de mise en oeuvre de l'invention dans lequel il y a trois électrodes tubulaires 4a, 4b, 4c disposées symétriquement autour d'une tuyère intermédiaire 24 fournissant un gaz intermédiaire $Q_3$. Ce dispositif permet de réaliser un plasma alimenté par courant triphasé. Les électrodes tubulaires sont inclinés vers l'intérieur, c'est-à-dire vers l'axe central du dispositif. Le plasma a la forme de trois jets 14a, 14b, 14c convergents sur une zone de traitement 16 de la décharge. Une telle décharge permet de concentrer dans une géométrie limitée une puissance électrique importante par rapport au cas de l'alimentation monophasée, et est moins susceptible de s'éteindre lors du contact avec la surface à traiter. Le débit du gaz d'apport $Q_1$ peut être important.

La fig. 5 montre une forme d'exécution du dispositif en peigne ou en brosse à plus de deux électrodes tubulaires 4a, 4b, 4c, 4d, 4e, 4f alimentées par du gaz d'apport $Q_1$ et de tuyères intermédiaires 24a, 24b, 24c, 24d, 24e, 24f alimentées par un gaz supplémentaire $Q_3$ disposées parallèlement les unes par rapport aux autres pour le traitement de larges surfaces, par exemple tissus biologiques, par exemple pour le traitement de surfaces de brûlures. Dans ce dernier cas il est possible grâce à ce dispositif d'effectuer le traitement en deux étapes, la première correspondante à l'arrêt d'épanchement (de suintement) et à l'assèchement de la surface à traiter, et la deuxième au dépôt d'un film de composition neutre, tel qu'un oxyde ou plus particulièrement un oxyde de silicium, ou d'un film organique, tel qu'un polyéthylène, remplissant le rôle de croûte obturant les microcanaux lymphatiques et protégeant la surface endommagée d'infections ou de contaminations. Le gaz supplémentaire $Q_3$ peut être différent du gaz d'apport $Q_1$ et, selon le traitement à effectuer, comprendre un mélange de gaz pour les réactions plasmo-chimiques voulues (telles que de l'hexaméthyldisilazane pour le dépôt d'un oxyde de silicium, etc.)

Dans la forme d'exécution de la fig. 5, grâce au petit diamètre des électrodes tubulaires, de préférence inférieures à 3 mm en diamètre, il est possible d'assurer la décharge électrique à des angles α = 0, de rapprocher les électrodes au maximum les uns des autres et, aussi, d'effectuer un traitement uniforme de la surface.

La fig. 6 montre un dispositif où la décharge est alimentée par un courant alternatif généré par un circuit d'alimentation selon la fig. 7b, et est comprimée contre la surface à traiter à l'aide d'un champ magnétique constant. La décharge vue dans le plan de la surface à traiter 19, ressemble à un papillon aux ailes écartées. La puissance électrique dégagée dans la décharge est distribuée sur toute la surface de la tâche. Cela permet d'effectuer le traitement de surfaces sensibles en régime de balayage assurant l'absence de zones brûlées localement. On peut obtenir une même sorte de décharge balayante en utilisant un courant continu et un champ magnétique alternatif.

Les figures 7a à 7e illustrent différents circuits d'alimentation de courant 12 de différents dispositifs mettant en oeuvre la présente invention, ainsi que les oscillogrammes du courant I de la décharge en fonction du temps t. Le circuit 12a de la fig. 7a correspond à une alimentation en courant continu à haute tension. La décharge se forme en courant continu à haute tension. La décharge se forme au claquage dans l'interstice entre les électrodes quand la tension aux bornes du condensateur C est assez élevée, et, après une certaine période de relaxation, le courant se stabilise. Ce circuit d'alimentation peut être utilisé dans le cas des dispositifs des figures 3, 13 et 14.

Le circuit 12b de la fig. 7b assure une alimentation en courant alternatif à haute tension. La décharge s'allume périodiquement et s'éteint à chaque demi-période de l'alimentation. La fréquence du processus peut être variée et contrôlée à l'aide du dispositif F. Les variations périodiques du courant s'accompagnent d'oscillations de fréquence sonique ou ultrasonique dans le plasma. Cela influence le caractère du traitement, par exemple, du tissus biologique. Ce circuit d'alimentation peut être utilisé dans les dispositifs illustrés aux figures 3, 7, 13 et 14 et est le seul possible pour le dispositif selon la fig. 6.

Le circuit 12c de la fig. 7c correspond à une alimentation par courant triphasé à haute tension (cas de la forme d'exécution fig. 4). Dans ce circuit d'alimentation, la décharge dans l'interstice entre les électrodes ne s'éteint jamais complètement, bien qu'elle disparaisse périodiquement entre les différentes paires concrètes d'électrodes. Cela assure la stabilité de la décharge, notamment quand le débit du gaz d'apport est important. Ce circuit permet d'actionner la décharge à tension plus faible que dans le cas d'une alimentation monophasée.

Le circuit d'alimentation 12d de la fig. 7d correspond à l'alimentation du dispositif par un courant triphasé avec bobines électriquement indépendantes. Le circuit peut être utilisé pour l'alimentation d'un dispositif avec un grand nombre d'électrodes, multiple de 3 (tel que la forme d'exécution de la fig. 5).

Le circuit d'alimentation 12e permet l'alimentation en impulsions du dispositif. Ce circuit permet d'assurer un traitement intense par exemple sur un tissus biologique, pendant de courts laps de temps avec des pauses de longue durée entre eux. Cela permet, entre autres, d'effectuer des traitements de surface sans exposer celle-ci à de grands flux (moyens) de chaleur. On peut régler des paramètres de décharge, tel que la durée entre les impulsions, leur intensité et la pente du front de croissance de l'impulsion.

La durée se règle par la grandeur de la résistance Ra qui détermine le temps de charge du condensateur C jusqu'à la tension de claquage. L'intensité des impulsions est déterminée par la capacité du condensateur C. L'énergie de l'impulsion, w, est égale à :

$$w = CU^2/2$$

où U est la tension aux bornes du condensateur. La pente de croissance se détermine par l'inductance L branchée consécutivement à la décharge.

Dans les circuits d'alimentation 12a, 12b, 12c et 12d, on utilise des résistances de fond inductives grâce auxquelles le flux de puissance active est limité et la stabilisation de la décharge est facilitée.

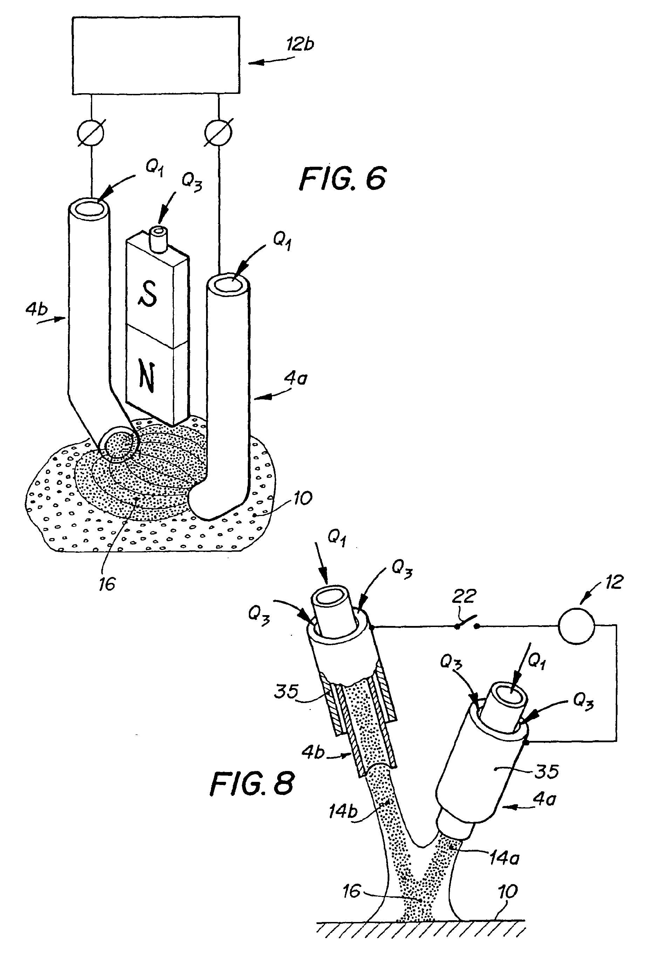

La fig. 8 illustre une forme d'exécution où les électrodes tubulaires 4a, 4b sont en matériaux diélectriques entourés de tubes 35 (en matériau conducteur, e.g. métallique) coaxiaux, alimentés par une source de courant haute fréquence et refroidis par un gaz $Q_3$ injecté dans un espace entre les tubes coaxiaux vers la zone de plasma 16.

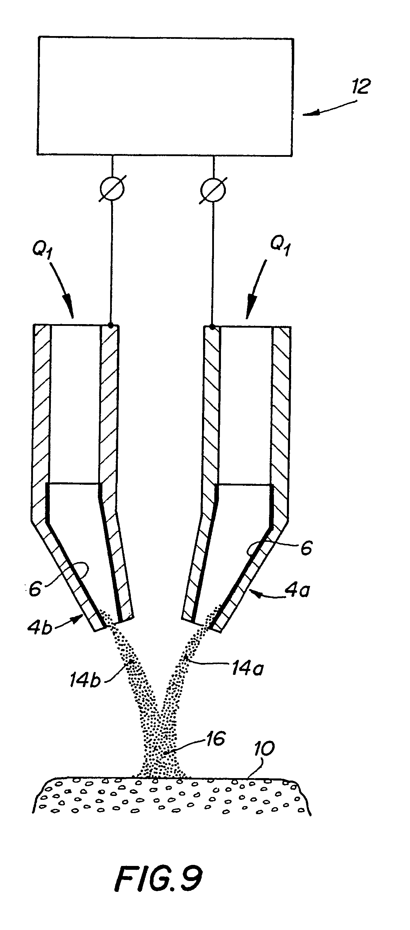

La fig. 9 illustre une forme d'exécution de l'invention où les électrodes tubulaires 4a, 4b sont exécutées sous forme de cônes confuseurs, ce qui permet d'augmenter la vitesse des flux des jets de plasma 14a, 14b et de mieux localiser la décharge de traitement 16 sur la surface à traiter.

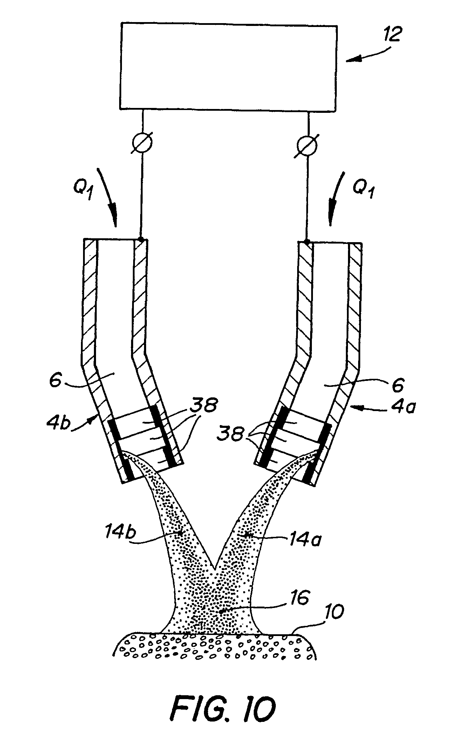

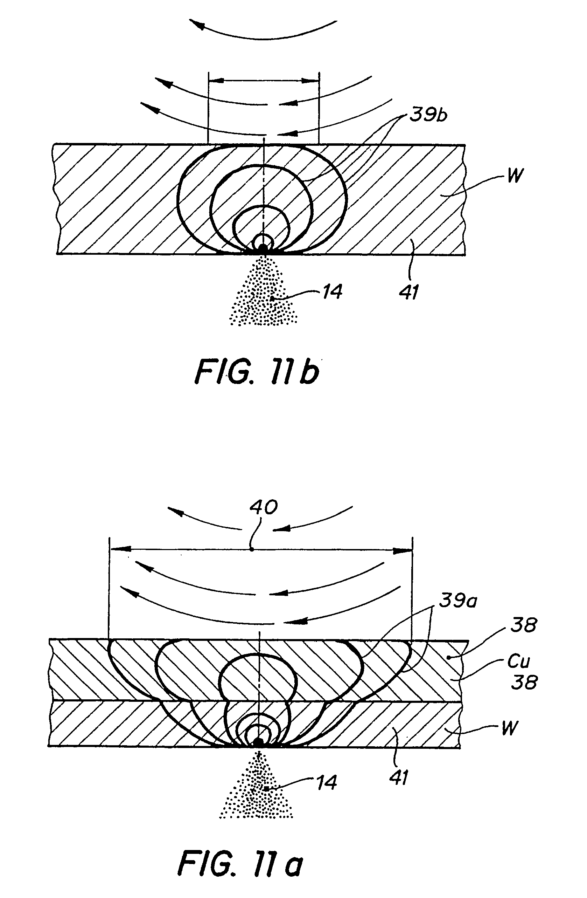

La fig. 10 illustre une forme d'exécution de l'invention où les électrodes tubulaires 4a, 4b sont pourvues de bagues 38 en métal de haute conductivité thermique (par exemple de Cu), ce qui permet, comme montré par les isothermes 39a dans la section de paroi d'électrode à la fig. 11a, d'agrandir la zone de refroidissement 40 des électrodes par rapport à une paroi d'électrode 41 sans bague, comme montré par les isothermes 39b dans la fig. 11b. La bague 38 assure un meilleur refroidissement tout en conservant le matériau de haute température de fusion (par exemple le tungstène W) pour assurer le fonctionnement électrodique en régime d'émission thermique.

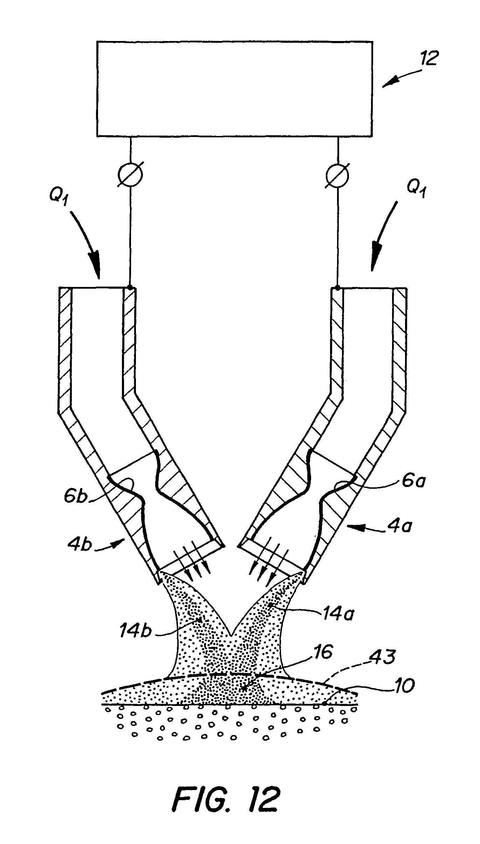

La fig. 12 illustre une forme d'exécution de la présente invention où les électrodes 4a, 4b tubulaires ont un canal d'écoulement de gaz d'apport $Q_1$ sous forme de tuyères supersoniques (tuyères de Laval). Ce mode est avantageux car il permet de projeter le plasma sur la surface avec une grande force hydrodynamique et d'exploiter l'onde de choc 43 qui se crée au-dessus de la surface à traiter 10, puisque celle-ci régénère le plasma 16 au contact même avec la surface à traiter.

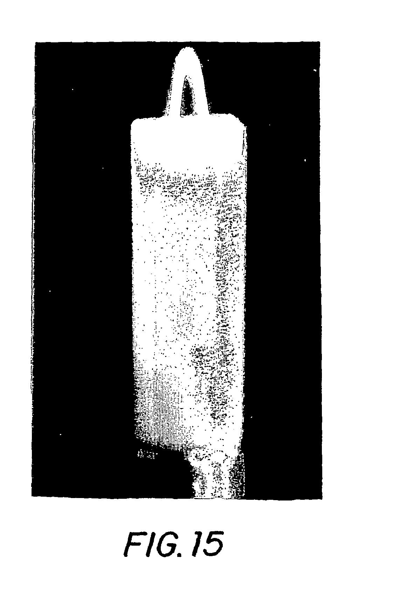

La fig. 15 est une photo de la décharge générée entre deux électrodes tubulaires par un dispositif similaire à celui montré dans la fig. 3a. Cette photo illustre la forme pointue du canal de plasma créé par la concurrence des forces hydrodynamiques et d'Ampère, d'une part, et des forces électrodynamiques assurant la décharge, d'autre part.

Les figures 16a à 16d sont des photos de décharges similaires à la fig. 15, illustrant différentes possibilités d'organisation de la décharge entre les électrodes tubulaires. Suivant la grandeur des flux de gaz alimentant les électrodes tubulaires, l'intensité du courant et la grandeur de la tension électrique, on peut réaliser une décharge diffuse entre les deux canaux issues des électrodes tubulaires, ceux-ci servant d'électrodes à cette décharge diffuse, comme montré dans la fig. 16a, ou une décharge de claquage entre ces canaux, comme montré dans la fig. 16b, ou des combinaisons de ces deux cas, comme montré dans les figures 16c et 16d.

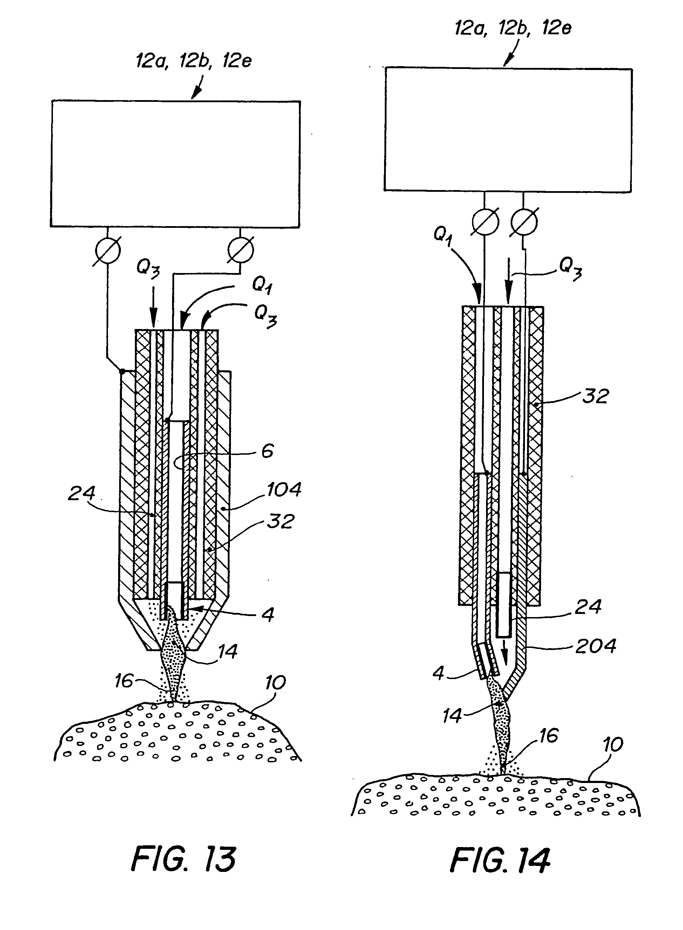

Une autre variante de mise en oeuvre de la présente invention est réalisée dans le dispositif illustré sur la fig. 13. Dans ce cas l'électrode intérieure 4, exécutée sous forme d'un tube fin, et l'électrode extérieure 104, exécutée sous forme d'un cône tronqué creux, sont disposés coaxialement. Le gaz d'apport $Q_1$ est introduit par un canal 6 dans l'électrode intérieure, mais peut également être introduit comme gaz supplémentaire $Q_3$ par des canaux 24, disposés symétriquement autour de l'électrode centrale. Dans une telle construction, le plasma a la forme d'un dard étroit qui permet d'effectuer un traitement local ponctuel par exemple sur un tissus biologique.

La fig. 14 représente un dispositif de mise en oeuvre de la présente invention dans lequel une des électrodes 204 est exécutée sous forme de tige monolithe en matériau réfractaire (par exemple en tungstène), dont l'extrémité est située sur la tangente au flux de gaz d'apport issu d'une électrode tubulaire 4. Une telle configuration permet de créer une décharge avec un dispositif de diamètre extérieur minimum, utile pour la coelioscopie, par exemple.

Exemple 1.

Activation d'une matière plastique

    Matière traitée : polyéthylène
    polypropylène
    polyéthylènethéréphtolate
    Source de courant : U = 1000 V, I = 100 mA
    Gaz d'apport : Kr (20 %) + $O_2$ (80 %)
    Traitement à l'air ambiant

Calcul de l'énergie superficielle $W_{surface}$

$$W_{surface} = c_{eau} (1 + \cos O)$$

$c_{eau}$ = 71,2 mJ/m$^2$
θ - angle de contact, degré

Les angles de contact θ sont mesurés par un appareil du type:Modèle CA-S-150 Digidrop.

Tableau 1 : Influence de la durée du traitement sur l'énergie superficielle de différentes matières plastiques

| N° | Durée (sec) | Energie superficielle 10$^{-3}$J/m2 (débit du gaz d'apport= 30l/min) | | |
|---|---|---|---|---|
| | | Polyéthylène | Polypropylène | Polyéthylènethéréphtolate |
| 1 | 0 | 77,3 | 81,2 | 82,5 |
| 2 | 3 | 118,9 | 129,7 | 142,1 |
| 3 | 5 | 124,3 | 131,8 | 142,5 |
| 4 | 10 | 135,1 | 137,7 | 142,7 |

(suite)

| N° | Durée (sec) | Energie superficielle $10^{-3}$ J/m2 (débit du gaz d'apport= 30l/min) | | |
|---|---|---|---|---|
| | | Polyéthylène | Polypropylène | Polyéthylènethéréphtolate |
| 5 | 15 | 139,7 | 140,1 | 142,5 |
| 6 | 20 | 139,4 | 139,6 | 142,6 |
| 7 | 25 | 138,7 | 139,1 | 142,3 |
| 8 | 40 | 131,1 | 122,5 | 142,1 |

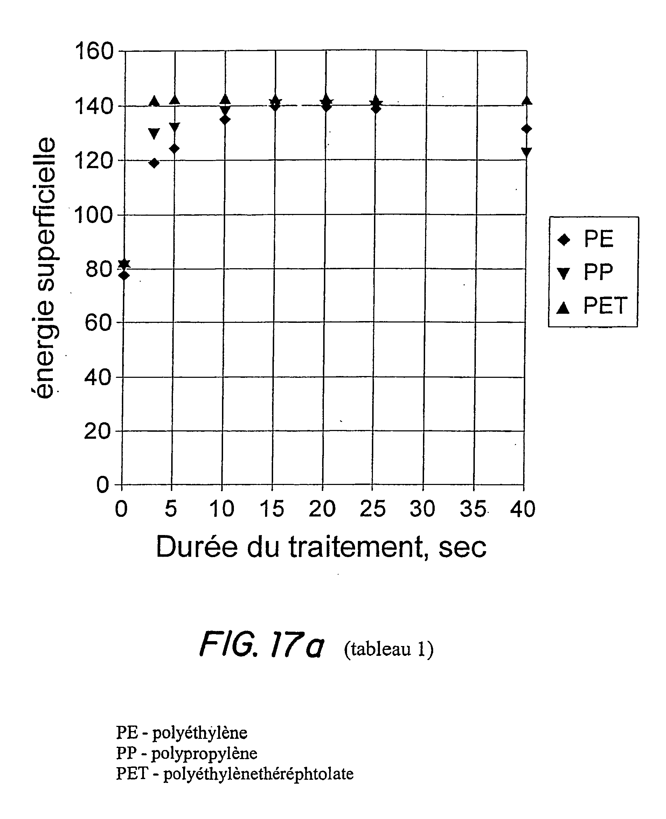

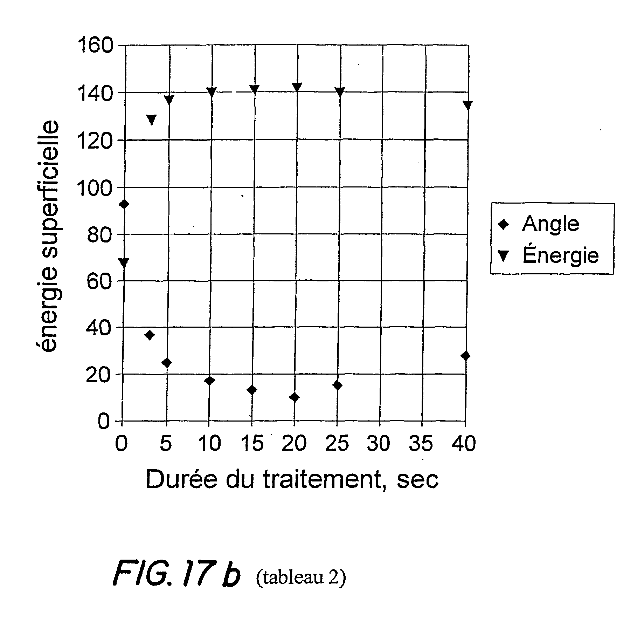

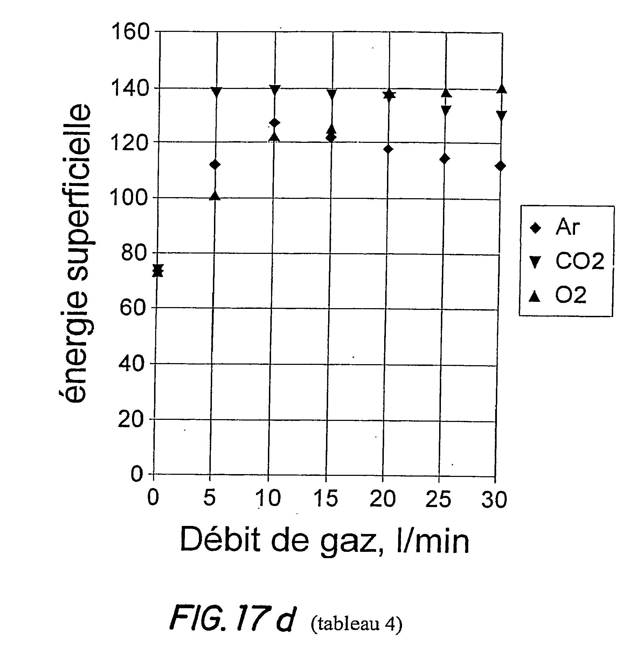

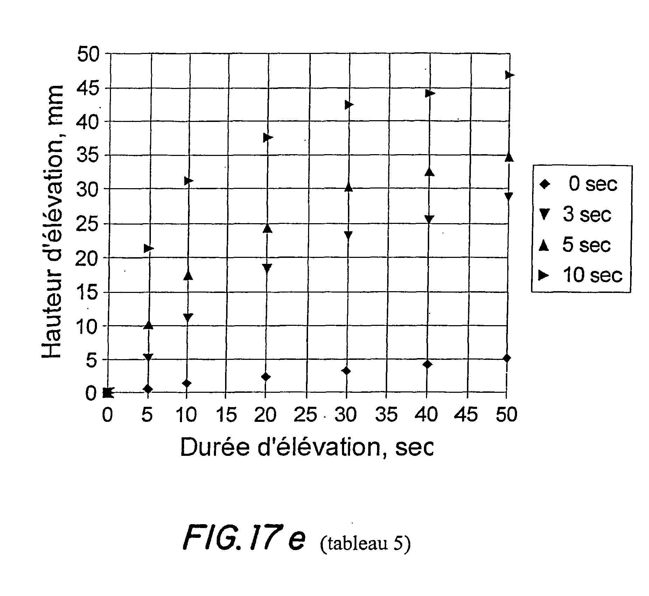

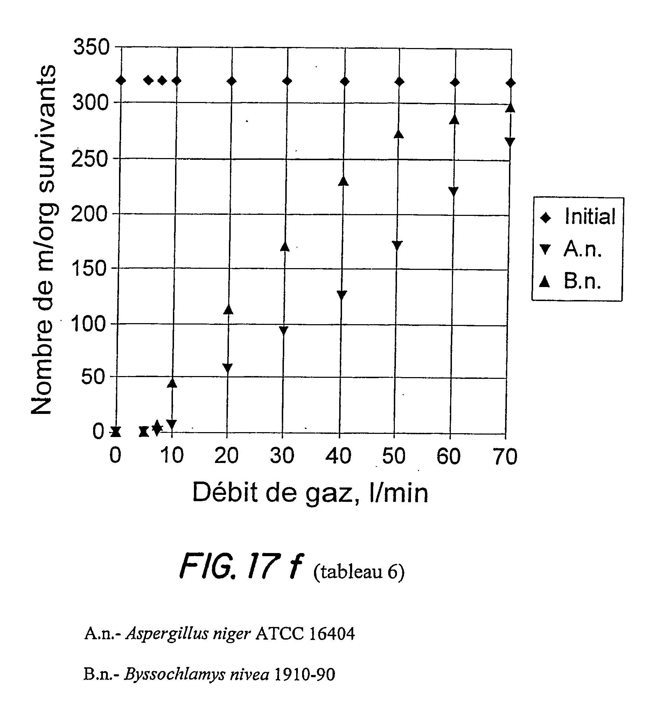

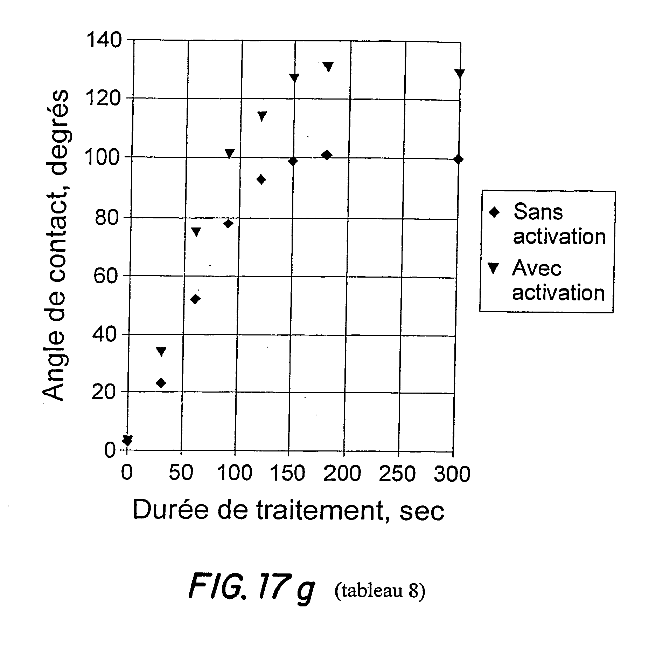

D'après les résultats ci-dessus on constante que l'activation de la matière plastique atteint un haut niveau après un traitement d'environ 5 sec pour toutes les matières plastiques qui sont utilisées. Les résultats du Tableau 1 sont illustrés graphiquement dans la fig. 17a.

Exemple 2 :

Activation de matière plastique avant peinture

Matière traitée: polypropylène
Source de courant: U = 100 V, I = 100 mA
Gaz d'apport: Xe (10%) + $O_2$ (90 %)
Traitement à l'air ambiant
Peinture: aérosol brillante

La mesure de l'adhésion de la peinture sur la matière plastique est réalisée suivant une méthode normalisée (standard 50488/01)

Tableau 2 : influence de la durée du traitement sur l'angle de contact, l'énergie superficielle et l'adhésion à la matière plastique.

| N° | Gaz | Débit de gaz d'apport (l/min) | Durée du traitement, (sec) | Diamètre de la goutte sur la surface, (mm) | Angle de contact, (degrés) | Energie superficielle (mJ/m$^2$) | Adhésion |
|---|---|---|---|---|---|---|---|
| 1 | $O_2$ | 30 | 0 | 3 | 93 | 67,6 | >Ad5 |
| 2 | $O_2$ | 30 | 3 | 5 | 37 | 128,2 | Ad 2 |
| 3 | $O_2$ | 30 | 5 | 6 | 25 | 136,1 | Ad 1 |
| 4 | $O_2$ | 30 | 10 | 8 | 17 | 139,6 | Ad 0 |
| 5 | $O_2$ | 30 | 15 | 11 | 13 | 140,5 | Ad 0 |
| 6 | $O_2$ | 30 | 20 | 15 | 10 | 141,3 | Ad 0 |
| 7 | $O_2$ | 30 | 25 | 10 | 15 | 139,8 | Ad 0 |
| 8 | $O_2$ | 30 | 40 | 7 | 28 | 133,8 | Ad 2 |

D'après les résultats ci-dessus on constate que l'activation provoque une importante adhésion. Les résultats du Tableau 2 sont illustrés graphiquement dans la fig. 17b.

Exemple 3.

Activation d'une matière plastique en utilisant des gaz d'apport différents

Matière traitée : polypropylène
Gaz : Ar (20 %) + (Ar, N$_2$, O$_2$, CO$_2$ ou Air) (80 %)
Source de courant : U = 100 V, I = 100 mA
Traitement à l'air ambiant

Tableau 3 : Influence de durée du traitement sur surface énergie de matière plastique pour différents gaz d'apport.

| N° | Durée (sec) | Energie superficielle, 10$^{-3}$J/m$^2$ (Polypropylène, Débit gaz = 30l/min) | | | | |
|---|---|---|---|---|---|---|
| | | N$_2$ | Ar | CO$_2$ | O$_2$ | Air |
| 1 | 0 | 73,7 | 73,7 | 73,7 | 73,7 | 73,7 |
| 2 | 3 | 105,1 | 93,4 | 105,7 | 129,8 | 122,8 |
| 3 | 5 | 121,1 | 106,8 | 118,9 | 135,2 | 127,6 |
| 4 | 10 | 122,9 | 113,9 | 130,3 | 140,3 | 131,9 |
| 5 | 15 | 119,2 | 122,2 | 130,3 | 141,3 | 125,5 |
| 6 | 20 | 107,7 | 123,2 | 129,5 | 141,1 | 123,3 |
| 7 | 30 | 101,3 | 119,8 | 130,2 | 141,7 | 114,3 |

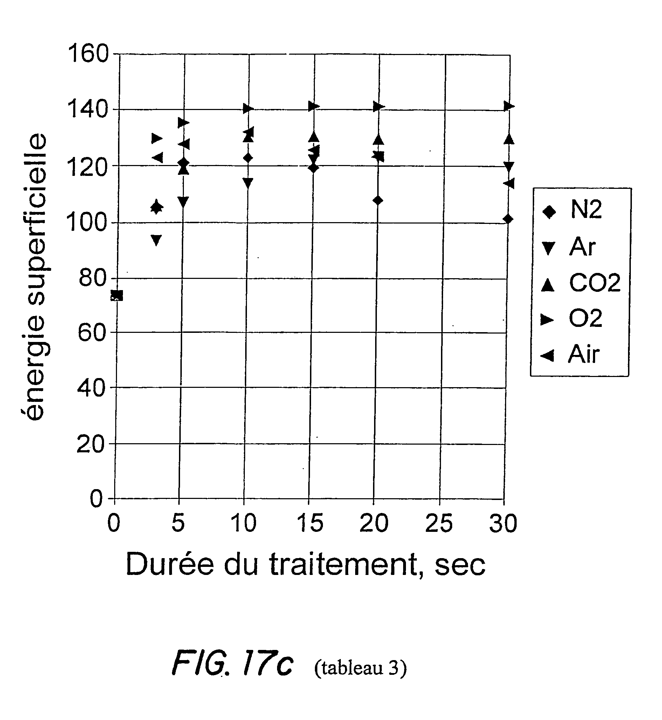

Les résultats du Tableau 3 sont illustrés graphiquement dans la fig. 17c.

Tableau 4 : Influence du débit de gaz sur l'énergie superficielle de la matière plastique traitée pour différents gaz d'apport.

| N° | Débit de gaz G/t (l/min) | Energie superficielle, 10$^{-3}$J/m$^2$ (durée du traitement 10 sec) | | |
|---|---|---|---|---|
| | | Ar | CO$_2$ | O$_2$ |
| 1 | 0 | 73,7 | 73,7 | 73,7 |
| 2 | 5 | 112,3 | 138,3 | 101,1 |
| 3 | 10 | 127,3 | 139,1 | 122,4 |
| 4 | 15 | 122,2 | 137,5 | 125,5 |
| 5 | 20 | 117,9 | 136,2 | 138,8 |
| 6 | 25 | 114,5 | 131,9 | 138,8 |
| 7 | 30 | 112,1 | 130,2 | 140,5 |

D'après les résultats ci-dessus on constate que différents gaz d'apport peuvent être utilisés pour l'activation de surface. Les meilleurs résultats sont obtenus avec l'oxygène. Les résultats du Tableau 4 sont illustrés graphiquement dans la fig. 17d.

Exemple 4 : Modification de la surface d'un tissu

Matière traitée : tissu (polyester) avec densité 820 g/m$^2$
Source de courant : U = 1000 V, I = 100 mA
Gaz d'apport: Ar (10 %) + oxygène (90 %)

L'activation d'obtenir en mesurant la vitesse d'élévation de l'eau (égale au rapport de la hauteur à la durée d'élévation) sur des échantillons orientés verticalement

Tableau 5 : Influence de durée du traitement sur la vitesse d'élévation de l'eau

| N° | Durée d'élévation (sec) | Hauteur d'élévation (mm) | | | |
|---|---|---|---|---|---|
| | | Durée du traitement 0 sec | Durée du traitement 3 sec | Durée du traitement 5 sec | Durée du traitement 10 sec |
| 1 | 0 | 0 | 0 | 0 | 0 |
| 2 | 5 | 0,5 | 5,1 | 10,2 | 21,3 |
| 3 | 10 | 1,4 | 11,2 | 17,5 | 31,2 |
| 4 | 20 | 2,3 | 18,3 | 24,4 | 37,5 |
| 5 | 30 | 3,2 | 23,2 | 30,3 | 42,4 |
| 6 | 40 | 4,1 | 25,4 | 32,5 | 44,1 |
| 7 | 50 | 5,2 | 28,7 | 34,7 | 46,8 |

On constate que la modification de la surface augmente sensiblement la vitesse de l'élévation. L'accentuation de propriétés hydrophiles contribue à l'amélioration de l'imprégnation. Les résultats du Tableau 5 sont illustrés graphiquement dans la fig. 17e.

Exemple 5 : Stérilisation d'échantillons contaminés par différents types de micro-organismes

Récipient : matière plastique - polypropylène
Source de courant : U = 1000 V, 1 =100 mA
Gaz : Ar(10%) + Air(90%)
Micro-organismes: *Aspergillus niger* ATCC 16404
*Byssochlamys nivea* 1910-90

La méthode de comptage du nombre de micro-organismes survivants a été effectuée selon des méthodes standards utilisées dans le domaine de la microbiologie.

Tableau 6 : Influence de débit de gaz sur nombre de micro-organismes survivants.

| N° | Débit (l/min) | Nombre de micro-organismes survivants | | |
|---|---|---|---|---|
| | | Initial Initial | Aspergillus niger ATCC 16404 | Byssochlamys nivea 1910-90 |
| 1 | 0 | 320 | - | - |
| 2 | 5 | 320 | 0 | 0 |
| 3 | 7,5 | 320 | 0 | 6 |
| 4 | 10 | 320 | 6 | 45 |
| 5 | 20 | 320 | 58 | 113 |
| 6 | 30 | 320 | 93 | 171 |
| 7 | 40 | 320 | 126 | 231 |
| 8 | 50 | 320 | 171 | 273 |
| 9 | 60 | 320 | 221 | 287 |
| 10 | 70 | 320 | 265 | 298 |

On constate que la stérilisation de la surface de l'échantillon est complète à des débits de gaz entre 5 et 7,5 l/min. Ce débit dépend du type de micro-organismes. Les résultats du Tableau 6 sont illustrés graphiquement dans la fig. 17f.

Exemple 6 : Modification d'une matière céramique et polymérisation de film sur la surface d'échantillons

Matière traité : matière céramique (par exemple tuile)
Source de courant : U = 1000 V, I = 100 mA
Gaz pour modification : Ar (20 %) + ($O_2$, $O_2$ + $CF_4$, air)(80 %)
Gaz pour polymérisation : Ar(30 %) + $C_3F_6$(70 %)
Liquide pour mesure d'angle de contact : eau, huile, pétrole

Les angles de contact sont mesurés par un appareil du type: Modèle CA-S-150 Digidrop

Tableau 7 : Influence de l'activation et de la polymérisation sur l'angle de contact

| N° | Activation | | Polymérisation | | Angle de contact (degrés) | | |
|---|---|---|---|---|---|---|---|
| | Gaz | Durée (sec) | Gaz | Durée (sec) | Eau | Huile | Pétrole |
| 1 | - | - | - | - | 55 | 43 | 44 |
| 2 | $O_2$ | 30 | - | - | 57 | 47 | 42 |
| 3 | $O_2$+$CF_4$ | 30 | - | - | 58 | 11 | 8 |
| 4 | Air | 30 | - | - | 7 | 6 | 0 |
| 5 | - | - | Ar+$C_3F_6$ | 300 | 149 | 111 | 85 |
| 6 | $O_2$ | 30 | Ar+$C_3F_6$ | 180 | 159 | 151 | 71 |
| 7 | $O_2$+$CF_4$ | 30 | Ar+$C_3F_6$ | 180 | 144 | 136 | 80 |
| 8 | Air | 30 | Ar+$C_3F_6$ | 180 | 149 | 142 | 90 |
| 9 | Air | 30 | Ar+$C_3F_6$ | 300 | 149 | 142 | 93 |

D'après les résultats ci-dessus, on constate que la polymérisation permet d'obtenir un film hydrophobe sur la surface d'échantillons céramique. La modification de surface avant polymérisation améliore les résultats de la polymérisation.

Exemple 7: Modification de surface et polymérisation de film sur la surface d'échantillons

Matière traitée : tissu en polyester (densité 450 g/m$^2$)
Source de courant : U = 1000 V, I = 100 mA
Gaz pour modification : Ar (10 %) + $O_2$ (90 %)
Gaz pour polymérisation : Ar (30 %) + $C_3F_6$ (70 %)
Liquide pour mesure d'angle de contact : eau

Les angles de contact sont mesurés par la méthode (Modèle CA-S-150 Digidrop)

Tableau 8 : Influence de l'activation et de la polymérisation sur l'angle de contact

| N° | Durée de polymérisation (sec) | Angle de contact (degrés) | | Energie superficielle (mJ/m$^2$) | |
|---|---|---|---|---|---|
| | | Sans activation | Avec activation | Sans activation | Avec activation |
| 1 | 0 | 3 | 3 | 142,3 | 142,3 |
| 2 | 30 | 23 | 34 | 136,8 | 130,2 |
| 3 | 60 | 52 | 75 | 115,1 | 89,7 |
| 4 | 90 | 78 | 101 | 85,9 | 57,7 |
| 5 | 120 | 93 | 114 | 67,5 | 42,2 |

(suite)

| N° | Durée de polymérisation (sec) | Angle de contact (degrés) | | Energie superficielle (mJ/m$^2$) | |
|---|---|---|---|---|---|
| | | Sans activation | Avec activation | Sans activation | Avec activation |
| 6 | 150 | 99 | 127 | 60,1 | 28,3 |
| 7 | 180 | 101 | 131 | 57,7 | 24,5 |
| 8 | 300 | 100 | 129 | 57,9 | 26,4 |

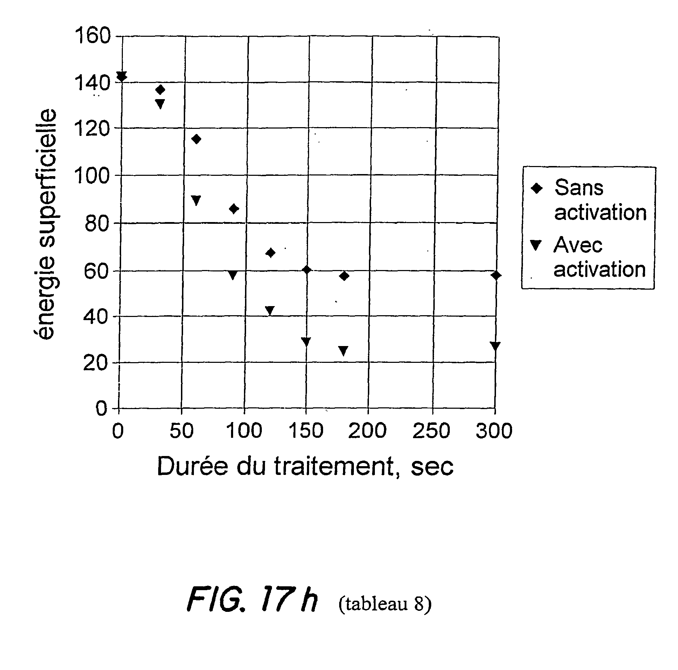

Les résultats ci-dessus montrent que la polymérisation permet d'obtenir un tissu avec des propriétés hydrophobes. L'activation effectuée avant la polymérisation améliore les propriétés hydrophobes. Les résultats du Tableau 8 sont illustrés graphiquement dans les figures 17g et 17h.

Dans tous les exemples cités, on a utilisé un générateur de plasma dont les éléments principaux sont des électrodes tubulaires alimentées par un mélange gazeux d'apport dont le potentiel d'ionisation et l'enthalpie d'ionisation sont inférieurs à ceux du gaz ambiant (de l'air ambiant).

Exemple 8 : Le dispositif utilisé dans cet exemple correspond au dispositif montré dans les photos des figures 15 et 16, correspondant essentiellement à la forme d'exécution de la fig. 3a, mais avec des électrodes tubulaires parallèles.

Le gaz d'apport, dans ce cas particulier est le krypton. Le dispositif plasma fonctionne dans l'air ambiant. On distingue dans les photos des figures 15 et 16nettement les deux électrodes tubulaires, ici, parallèles l'une à l'autre. De ces électrodes sont projetés deux jets brillants parallèles de plasma. L'espace entre ces jets, nettement plus sombre, correspond à la zone, traversée par les électrons, où le plasma est hors d'équilibre thermodynamique et chimique. Les électrons ont une haute énergie et leur longueur de parcours moyen est élevé, atteignant plusieurs millimètres. Ce n'est que dans les zones des jets qu'ils perdent leur énergie cinétique sous forme d'énergie d'excitation du gaz issu des jets. Les deux canaux de plasma visibles sont formés par le courant d'électrons ionisant le gaz d'apport éjecté des électrodes tubulaires, ce gaz ayant une énergie d'ionisation inférieure à celle du gaz ambiant (l'air dans ce cas).

Les deux jets de plasma constituent en somme deux électrodes de plasma entre lesquelles a lieu la décharge électrique. Celle-ci, suivant le débit de gaz d'apport par les électrodes tubulaires, et la tension entre ces électrodes peut avoir la forme d'une décharge diffuse, non autonome, ou d'un claquage avec formation de filaments localisés de plasma.

Exemple 9 : Utilisation du du dispositif de traitement de surface selon l'invention pour les interventions chirurgicales :

1. Cautérisation des effusions sanguines
2. Arrêt des suintements de plasma sanguin dans les brûlures

1. Cautérisation des effusions sanguines.

Des opérations chirurgicales ont été effectuées sur des porcs. Le dispositif utilisé comprend un générateur de plasma à deux électrodes tubulaires, comme dans l'exemple 8 ci-dessus, par lesquelles étaient introduits des gaz (Ar, Kr, Xe) dont l'énergie d'ionisation (enthalpie d'ionisation est inférieure à celle de l'air ambiant). Dans la zone de traitement étaient introduits des gaz tels que $O_2$, $CO_2$, $N_2$ par une tuyère intermédiaire. La fréquence de génération était 300kHz. L'amplitude de la tension électrique était de 300V. Le débit de gaz introduit par les électrodes tubulaires variait entre 0,11/min et 2l/min. Le débit des gaz additionnels variait entre 0,2l/min et 3l/min.

Les opérations étaient effectuées à ciel ouvert et en coelioscopie. Dans ce dernier cas, l'appareil contenant le générateur de plasma était équipé d'une tubulure avec autorégulation de pression de manière à empêcher la pression à l'intérieur de l'enceinte opératoire de croître pendant l'utilisation du générateur de plasma. Le but de l'opération était de tester la capacité de cautérisation à l'aide du dispositif mentionné. Des hémorragies abondantes ont été , à cet effet, occasionnées sur la peau, le muscle, le diaphragme, le foie, la rate, l'intestin, la vésicule biliaire, la vessie, les organes génitaux internes, des vaisseaux (veines et même petites artères, le poumon, le coeur). Dans tous les cas, l'appareil a permis la cautérisation rapide et efficace des hémorragies.

Les études histologiques post-opératoires ont montré que les dommages sur les tissus traités, étaient beaucoup moins importants que dans le cas de l'utilisation d'un cautériseur à une électrode (du type Erbotom 12C350, qui était utilisé en parallèle avec le présent dispositif pour comparaison), ce qui est dû à l'absence du passage destructif du courant électrique par la plaie à travers le patient.

2. <u>Arrêt des suintements de plasma sanguin après brûlures.</u>

Des opérations ont été effectuées sur des porcs dont la surface du dos avait été brûlée au 3ième degré. A l'aide de l'appareil sus-mentionné dans les mêmes conditions, il a été possible de cautériser les infiltrations (suintements) de plasma de sang caractéristiques de ce type de brûlure.

Les zones brûlées se sont rapidement séchées et guéries. Aucune infection n'a été relevée, bien que la brûlure traitée n'ait pas été spécialement protégée de contamination. Cela signifie que le traitement de la brûlure par le générateur de plasma à deux électrodes tubulaires est non seulement un traitement cautérisant mais, en plus, un traitement stérilisant.

Un traitement de "caramélisation" a été également effectué sur des brûlures du 3ième degré. Dans ce cas, en matière de gaz additif, on a utilisé un mélange d'argon, d'héxaméthyldisilazane et d'oxygène. La zone de brûlure a préalablement été séchée par un plasma d'argon et d'oxygène, ensuite a été recouverte d'une mince croûte (~ 0,1μm) d'oxyde de silicium, qui a joué le rôle de protection imperméable aux éléments contaminants extérieurs. Vu la minceur de cette couche, elle n'a occasionné aucune blessure postérieure et a disparu sans traces, laissant une cicatrice propre, disparaissant rapidement, elle aussi.

**Revendications**

1.  Procédé de traitement de surface par plasma atmosphérique comprenant la génération d'au moins un jet de plasma par un générateur de plasma, le jet de plasma étant créé par une décharge électrique dans des flux de gaz ou de mélange gazeux d'apport ayant une enthalpie d'ionisation inférieure à celle du milieu gazeux ambiant, **caractérisé en ce qu'**une zone de la décharge électrique disposée entre ledit jet de plasma qui agit comme électrode, et une deuxième électrode ou jet de plasma agissant comme électrode, est non-autonome et génère un plasma formé principalement d'un gaz activé servant à traiter la surface, l'intensité E du champ électrique créant la décharge répondant à la condition :

$$(JnQ/e)_{\text{gaz d'apport}} \le E \le (JnQ/e)_{\text{gaz ambiant}}$$

où J est l'énergie d'activation des particules du gaz
n - la densité des particules de ce gaz
Q - la section efficace des collisions élastiques des électrons avec les particules de ce gaz
e - la charge de l'électron.

2.  Procédé selon la revendication 1, **caractérisé en ce que** l'on génère au moins deux jets de plasma agissant comme électrodes, par des générateurs des plasma.

3.  Procédé selon la revendication 2, **caractérisé en ce que** l'on génère les jets de plasma à partir d'électrodes tubulaires ayant des canaux dans lesquels on injecte le gaz d'apport.

4.  Procédé selon la revendication 2 ou 3, **caractérisé en ce que** l'on génère des claquages entre les jets de plasma.

5.  Procédé selon les revendications 2 à 4, **caractérisé en ce que** l'on applique un champ magnétique, dirigé essentiellement perpendiculairement à la surface à traiter pour élargir la zone d'action du plasma sur la surface à traiter.

6.  Procédé selon l'une des revendications 2 à 5, **caractérisé en ce que** les jets de plasma sont dirigés de manière réglable pour créer une zone de traitement soit localisée soit large, cette zone étant comprimée contre la surface à traiter simultanément par les forces d'inertie hydrodynamique et les forces d'Ampère.

7.  Procédé selon l'une des revendications 2 à 6, **caractérisé en ce que** les axes des électrodes sont parallèles l'un à l'autre.

8.  Procédé selon la revendication 1, **caractérisé en ce que** l'on génère au moins un jet de plasma par un générateur de plasma comportant une électrode tubulaire ayant un canal dans lequel on injecte un gaz d'apport, et une deuxième électrode.

9. Procédé selon l'une des revendications précédentes, **caractérisé en ce que** le plasma servant à traiter la surface est formé dans un gaz ou un mélange gazeux $Q_2$, $Q_3$, différent du gaz ou mélange gazeux d'apport $Q_1$.

10. Procédé selon l'une des revendications précédentes, **caractérisé en ce que** le plasma est alimenté par impulsions électriques unipolaires ou bipolaires dont on règle la durée du front, la durée de l'impulsion et le laps de temps entre les impulsions.

11. Dispositif pour le traitement de surface par plasma atmosphérique selon la revendication 1, **caractérisé en ce qu'**il comporte au moins deux électrodes, une des électrodes étant sous forme de tube formant un canal central d'écoulement du gaz d'apport alimenté par un système d'alimentation en gaz d'apport, les électrodes étant connectées à un circuit d'alimentation de courant apte à gérer une intensité E du champ électrique créant une décharge répondant à la condition :

$$(JnQ/e)_{\text{gaz d'apport}} \leq E \leq (JnQ/e)_{\text{gaz ambiant}}$$

où J est l'énergie d'activation des particules du gaz
n - la densité des particules de ce gaz
Q - la section efficace des collisions élastiques des électrons avec les particules de ce gaz
e - la charge de l'électron,
et un système d'alimentation en gaz d'apport, chaque électrode sous forme de tube formant un canal central d'écoulement du gaz d'apport.

12. Dispositif pour le traitement de surface par plasma atmosphérique selon la revendication 1, **caractérisé en ce qu'**il comporte au moins deux électrodes sous forme de tubes dont les axes se recoupent sous un angle pouvant varier entre 0° et 180°, auxquels sont connectés un circuit d'alimentation de courant apte à gérer une intensité E du champ électrique créant une décharge répondant à la condition :

$$(JnQ/e)_{\text{gaz d'apport}} \leq E \leq (JnQ/e)_{\text{gaz ambiant}}$$

où J est l'énergie d'activation des particules du gaz
n - la densité des particules de ce gaz
Q - la section efficace des collisions élastiques des électrons avec les particules de ce gaz
e - la charge de l'électron.

13. Dispositif selon la revendication 12, **caractérisé en ce que** le point d'intersection des axes des électrodes tubulaires se trouve en-dessous de la surface à traiter, ce qui oblige les flux de plasma issus des électrodes, de longer la surface à traiter.

14. Dispositif selon la revendication 12, **caractérisé en ce que** un ou plusieurs des flux de gaz sont dirigés par l'intermédiaire de tuyères cylindriques ou aplaties disposées entre les électrodes tubulaires vers la surface à traiter, de manière à modifier la composition du gaz activé contre la surface à traiter.

15. Dispositif selon la revendication 12, **caractérisé en ce qu'**une pluralité de paires d'électrodes tubulaires sont disposées en ligne de manière à former un peigne, permettant de balayer une grande surface de l'objet à traiter.

16. Dispositif selon l'une des revendications précédentes, comprenant des moyens de réglage des angles entre les électrodes et de la distance entre les électrodes.

17. Dispositif selon l'une des revendications précédentes, comprenant des moyens de réglage du débit des gaz introduits à travers les électrodes tubulaires du débit des gaz introduits entre les jets de plasma du courant et de la tension entre les électrodes.

18. Dispositif selon l'une des revendications précédentes, comprenant des moyens de réglage de la distance des électrodes à la surface du matériau à traiter.

**19.** Dispositif selon la revendication 12, **caractérisé en ce que** les électrodes tubulaires sont disposées de manière à ce que leurs axes soient parallèles.

**20.** Dispositif selon la revendication 12, comprenant un système de tubes longitudinaux disposés en nids d'abeilles, les tubes étant de longueur variable destinés à profiler la distribution des vitesses d'écoulement du gaz d'apport afin d'éviter une turbulisation du flux de plasma issu des électrodes.

**21.** Dispositif selon la revendication 12, comprenant une tuyère avec des perforations radiales disposée entre les électrodes pour assurer que la distribution du flux de gaz injecté entre les électrodes tubulaires se fait radialement et dans la direction des jets de plasma.

**22.** Dispositif selon la revendication 12, **caractérisé en ce qu'**il comporte trois électrodes tubulaires alimentées par une source de courant triphasé, le gaz d'apport étant amené par l'intermédiaire d'un tube, coaxial, et équidistant de ceux-ci.

**23.** Dispositif selon la revendication 12, **caractérisé en ce que** entre les électrodes est disposé un générateur de champ magnétique, refroidi, créant un champ perpendiculaire à la surface à traiter.

**24.** Dispositif selon la revendication 11 ou 12, **caractérisé en ce que** la source de courant pour l'alimentation de la décharge électrique est une source de courant continu.

**25.** Dispositif selon la revendication 11 ou 12, **caractérisé en ce que** la source de courant pour l'alimentation de la décharge électrique est une source de courant alternatif triphasé.

**26.** Dispositif selon la revendication 11 ou 12, **caractérisé en ce que** la source de courant pour l'alimentation de la décharge électrique est une source d'impulsions dont le front, la durée, et la cadence sont réglables.

**27.** Dispositif selon la revendication 11 ou 12, **caractérisé en ce que** la ou les électrodes tubulaires sont exécutées sous forme de tubes en matériau diélectrique entourés de tubes métalliques coaxiaux, alimentés par une source de courant à haute fréquence refroidis par un gaz dirigé vers la zone de plasma.

**28.** Dispositif selon la revendication 11 ou 12, **caractérisé en ce que** la ou les électrodes tubulaires sont exécutées sous forme de tubes coniques, pour localiser les flux de gaz d'apport sur la zone à traiter.

**29.** Dispositif selon la revendication 11 ou 12, **caractérisé en ce que** la ou les électrodes tubulaires sont refroidies extérieurement et équipées intérieurement de bagues tubulaires de manière à dissiper le flux de chaleur émis dans les taches d'électrodes.

**30.** Dispositif selon la revendication 11 ou 12, **caractérisé en ce que** la ou les électrodes tubulaires sont exécutées en forme de tuyères supersoniques ou de tuyères de Laval, de manière à augmenter les forces hydrodynamiques de compression du plasma sur la surface à traiter, en créant au-dessus de la surface à traiter une onde de choc.

**31.** Dispositif selon la revendication 11, **caractérisé en ce que** la deuxième électrode est sous forme de cône tronqué creux disposé coaxialement autour de l'électrode tubulaire.

**32.** Dispositif selon la revendication 11, **caractérisé en ce que** la deuxième électrode est sous forme de tige.

**33.** Utilisation d'un dispositif selon l'une des revendications précédentes pour le traitement des matériaux textiles pour leur donner des propriétés hydrophiles ou hydrophobes.

**34.** Utilisation d'un dispositif selon l'une des revendications précédentes pour le traitement de matériaux céramiques pour en assurer des propriétés hydrophiles ou hydrophobes.

**35.** Utilisation d'un dispositif selon l'une des revendications précédentes pour la cautérisation du sang et tissus médico-biologiques lors d'opérations chirurgicales.

**36.** Utilisation d'un dispositif selon l'une des revendications précédentes pour l'arrêt des suintements de liquide lymphatique lors de brûlures.

37. Utilisation d'un dispositif selon l'une des revendications précédentes pour l'activation de surface d'objets métalliques, faiblement conducteurs, et diélectrique, pour en augmenter l'adhésion à la peinture, à la colle, ou à d'autres substances.

**Claims**

1. Method of treating a surface by atmospheric plasma, including the generation of at least one plasma jet using a plasma generator, the plasma jet being created by an electric discharge in streams of a carrier gas or of a carrier gas mixture having an ionisation enthalpy lower than that of the ambient gaseous medium, **characterized in that** a zone of electric discharge located between said plasma jet which functions as an electrode, and a second electrode or a plasma jet functioning as an electrode, is non-autonomous and generates a plasma comprised mainly of an activated gas used for treating the surface, the intensity E of the electric field creating the discharge satisfying the condition :

$$(JnQ \,/\, e)_{\text{carrier gas}} \leq E \leq (JnQ \,/\, e)_{\text{ambient gas}}$$

in which J is the energy of activation of the gas particles
n - the density of the particles of this gas
Q - the effective cross-section of the elastic collisions of the electrons with the particles of this gas
e - the charge of an electron.

2. Method according to claim 1, **characterized in that** at least two plasma jets are generated, which function as electrodes, by using plasma generators.

3. Method according to claim 2, **characterized in that** the plasma jets are generated from tubular electrodes having channels into which is injected the carrier gas.

4. Method according to claim 2 or 3, **characterized in that** electric breakdowns are generated between the plasma jets.

5. Method according to claims 2 to 4, **characterized in that** a magnetic field is applied, which is directed substantially perpendicularly to the surface to be treated, in order to broaden the zone of action of the plasma on the surface to be treated.

6. Method according to one of claims 2 to 5, **characterized in that** the plasma jets are directed adjustably, to create a treatment zone which is either confined or broad, this zone being urged against the surface to be treated simultaneously by inertial hydrodynamic forces and by Ampere's forces.

7. Method according to one of claims 2 to 6, **characterized in that** the axes of the electrodes are parallel to each other.

8. Method according to claim 1, **characterized in that** at least one plasma jet is generated by a plasma generator comprising a tubular electrode having a channel into which a carrier gas is injected and a second electrode.

9. Method according to one of the preceding claims, **characterized in that** the plasma for treating the surface is formed in a gas or in a gaseous mixture $Q_2$, $Q_3$, which differs from the carrier gas or the carrier gas mixture $Q_1$.

10. Method according to one of the preceding claims, **characterized in that** the plasma is generated by unipolar or bipolar electric pulses, wherein the duration of the leading edge of the pulses, the duration of the pulses and the time elapsed between the pulses are adjusted.

11. Device for the treatment of a surface by atmospheric plasma according to claim 1, **characterized in that** it includes at least two electrodes, one of the electrodes provided as a tube forming a central flow channel for a carrier gas supplied from a carrier gas supply system, the electrodes being connected to a power supply circuit adapted to control an electric field creating a discharge having an intensity E satisfying the condition :

$$(JnQ / e)_{carrier\ gas} \leq E \leq (JnQ / e)_{ambient\ gas}$$

in which J is the energy of activation of the gas particles

n - the density of the particles of this gas

Q - the effective cross-section of the elastic collisions of the electrons with the particles of this gas

e - the charge of an electron,

and a carrier gas supply system, each electrode being provided as a tube forming a central flow channel for the carrier gas.

12. Device for the treatment of a surface by atmospheric plasma according to claim 1, **characterized in that** it includes at least two electrodes provided as tubes having axes intersecting at an angle in the range from 0 ° to 180 ° and to which is connected a power supply circuit adapted to control an electric field creating a discharge having an intensity E satisfying the condition :

$$(JnQ / e)_{carrier\ gas} \leq E \leq (JnQ / e)_{ambient\ gas}$$

in which J is the energy of activation of the gas particles

n - the density of the particles of this gas

Q - the effective section of the elastic collisions of the electrons with the particles of this gas

e - the charge of an electron.

13. Device according to claim 12, **characterized in that** the point of intersection of the axes of the tubular electrodes is located beneath the surface to be treated, which forces the streams of plasma emitted from the electrodes to flow along the surface to be treated.

14. Device according to claim 12, **characterized in that** one or several of the gas streams are directed from cylindrical or flattened nozzles, positioned between the tubular electrodes, in the direction of the surface to be treated, in such a manner as to modify the composition of the activated gas impinging upon the surface to be treated.

15. Device according to claim 12, **characterized in that** a plurality of pairs of tubular electrodes is arranged along a line in such a manner as to form a comb, allowing to sweep a broad surface of the object to be treated.

16. Device according to one of the preceding claims, including means for adjusting the angles between the electrodes and the distance between the electrodes.

17. Device according to one of the preceding claims, including means for adjusting the flow rate of the gas introduced via the tubular electrodes, of the flow rate of the gases introduced between the plasma jets, of the magnitude of the current and of the voltage drop between the electrodes.

18. Device according to one of the preceding claims, including means for adjusting the distance of the electrodes to the surface of the material to be treated.

19. Device according to claim 12, **characterized in that** the tubular electrodes are positioned in such a manner that their axes be parallel to each other.

20. Device according to claim 12, including a system of longitudinal tubes provided as a honeycomb, the tubes having a length which varies in order to confer a specific profile to the distribution of the flow velocity of the carrier gas, designed to avoid turbulence in the stream of the plasma emitted from the electrodes.

21. Device according to claim 12, including a nozzle with radial perforations, provided between the electrodes in order to ensure that the streams of the gas injected between the tubular electrodes are directed radially and in the direction of the plasma jets.

22. Device according to claim 12, **characterized in that** it includes three tubular electrodes supplied from a three-phase power supply source, the carrier gas being supplied via a coaxial tube which is equidistant therefrom.

23. Device according to claim 12, **characterized in that** between the electrodes, there is provided a cooled magnetic field generator, creating a field which is perpendicular to the surface to be treated.

24. Device according to claim 11 or 12, **characterized in that** the power supply source for producing the electric discharge is a direct current supply source.

25. Device according to claim 11 or 12, **characterized in that** the power supply source for producing the electric discharge is an alternating three-phase current supply source.

26. Device according to claim 11 or 12, **characterized in that** the power supply source for producing the electric discharge is a source generating pulses, of which the leading edge, the duration and the frequency are adjustable.

27. Device according to claim 11 or 12, **characterized in that** the tubular electrodes are provided as tubes made of a dielectric material surrounded by coaxial metal tubes, power supplied by a high frequency power supply source and cooled by a gas directed towards the plasma zone.

28. Device according to claim 11 or 12, **characterized in that** the tubular electrodes are provided as conical tubes, to focus the streams of the carrier gas on the zone to be treated.

29. Device according to claim 11 or 12, **characterized in that** the tubular electrodes are cooled externally and are equipped internally with tubular rings, in such a manner as to dissipate the heat produced by the electrode spots.

30. Device according to claim 11 or 12, **characterized in that** the tubular electrodes are provided as supersonic nozzles or Laval nozzles, in order to increase the hydrodynamic forces urging the plasma against the surface to be treated, by creating a shock wave above the surface to be treated.

31. Device according to claim 11, **characterized in that** the second electrode is provided as a hollow truncated cone arranged coaxially about the tubular electrode.

32. Device according to claim 11, **characterized in that** the second electrode is provided as a rod.

33. Use of a device according to one of the preceding claims, for the treatment of textile materials, in order to confer thereto hydrophilic or hydrophobic properties.

34. Use of a device according to one of the preceding claims, for the treatment of ceramic materials, in order to confer thereto hydrophilic or hydrophobic properties.

35. Use of a device according to one of the preceding claims, for the cauterisation of blood and of medico-biological tissues during surgical operations.

36. Use of a device according to one of the preceding claims, for stopping the oozing of lymphatic liquid from burns.

37. Use of a device according to one of the preceding claims, for the activation of the surface of metal objects, of feebly conductive objects and of dielectric objects, in order to increase their adhesivity to paints, to adhesives or to other substances.

**Patentansprüche**

1. Verfahren für die Oberflächenbehandlung durch ein atmosphärisches Plasma, die Erzeugung zumindest eines Plasmastrahls durch einen Plasmagenerator umfassend, wobei der Plasmastrahl durch eine elektrische Entladung in einem Strom von Zusatzgas oder Zusatzgasgemisch erzeugt wird, die eine geringere Ionisierungsenthalpie als das umgebende Gasmedium besitzen, **dadurch gekennzeichnet, dass** eine Zone der elektrischen Entladung, die zwischen dem als Elektrode wirkenden Plasmastrahl und einer zweiten Elektrode bzw. einem als Elektrode wirkenden zweiten Plasmastrahl angeordnet ist, unselbständig ist und ein Plasma erzeugt, das hauptsächlich aus einem aktivierten Gas gebildet wird, das dazu dient, die Oberfläche zu behandeln, wobei die Intensität E des elektrischen Feldes, das die Entladung erzeugt, der Bedingung gehorcht:

$$(J\,n\,Q\,/\,e)_{Zusatzgas} \leq E \ll (J\,n\,Q\,/\,e)_{Umgebungsgas},$$

wo

J die Aktivierungsenergie der Gasteilchen,
n die Dichte der Teilchen dieses Gases,
Q der Wirkungsquerschnitt der elastischen Zusammenstösse zwischen Elektronen und den Teilchen dieses Gases und
e die Elektronenladung ist.

2.  Verfahren nach Anspruch 1, **dadurch gekennzeichnet, dass** durch Plasmageneratoren zumindest zwei Plasmastrahlen erzeugt werden, die als Elektroden wirken.

3.  Verfahren nach Anspruch 2, **dadurch gekennzeichnet, dass** die Plasmastrahlen von rohrförmigen Elektroden ausgehend erzeugt werden, die Kanäle besitzen, in die das Zusatzgas injiziert wird.

4.  Verfahren nach Anspruch 2 oder 3, **dadurch gekennzeichnet, dass** Durchschläge zwischen den Plasmastrahlen erzeugt werden.

5.  Verfahren nach Ansprüchen 2 bis 4, **dadurch gekennzeichnet, dass** ein magnetisches Feld angelegt wird, das im Wesentlichen senkrecht auf die zu behandelnde Oberfläche gerichtet ist, um den Wirkungsbereich des Plasmas auf der zu behandelnden Oberfläche zu erweitern.

6.  Verfahren nach einem der Ansprüche 2 bis 5, **dadurch gekennzeichnet, dass** die Plasmastrahlen in regelbarer Weise ausgerichtet werden, um einen Behandlungsbereich zu schaffen, der entweder lokalisiert oder breit ist, wobei dieser Bereich gleichzeitig durch die hydrodynamischen Trägheitskräfte und die Amperekräfte gegen die zu behandende Oberfläche gedrückt wird.

7.  Verfahren nach einem der Ansprüche 2 bis 6, **dadurch gekennzeichnet, dass** die Achsen der Elektroden zueinander parallel sind.

8.  Verfahren nach Anspruch 1, **dadurch gekennzeichnet, dass** zumindest ein Plasmastrahl durch einen Plasmagenerator erzeugt wird, der eine rohrförmige Elektrode, die einen Kanal besitzt, in den ein Zusatzgas injiziert wird, sowie eine zweite Elektrode umfasst.

9.  Verfahren nach einem der vorangehenden Ansprüche, **dadurch gekennzeichnet, dass** das zur Behandlung der Oberfläche dienende Plasma in einem Gas oder einem Gasgemisch $Q_2$, $Q_3$ gebildet wird, das sich vom Zusatzgas bzw. Zusatzgasgemisch $Q_1$ unterscheidet.

10.  Verfahren nach einem der vorangehenden Ansprüche, **dadurch gekennzeichnet, dass** das Plasma durch unipolare oder bipolare elektrische Impulse betrieben wird, deren Anstiegszeit, Impulsdauer und zeitlicher Abstand zwischen den Impulsen geregelt wird.

11.  Vorrichtung zur Oberflächenbehandlung durch ein atmosphärisches Plasma nach Anspruch 1, **dadurch gekennzeichnet, dass** sie zumindest zwei Elektroden umfasst, wobei eine der Elektroden in Gestalt eines Rohres vorliegt, das einen zentralen Kanal für den Durchfluss des Zusatzgases bildet, der durch ein Versorgungssystem mit Zusatzgas versorgt wird, und wobei die Elektroden mit einem Stromversorgungskreis, der in der Lage ist, eine elektrische Feldintensität E aufrechtzuerhalten, die eine Entladung erzeugt, die der Bedingung gehorcht:

$$(J\,n\,Q\,/\,e)_{Zusatzgas} \leq E \ll (J\,n\,Q\,/\,e)_{Umgebungsgas},$$

wo

J die Aktivierungsenergie der Gasteilchen,

n die Dichte der Teilchen dieses Gases,

Q der Wirkungsquerschnitt der elastischen Zusammenstösse zwischen Elektronen und den Teilchen dieses Gases und

e die Elektronenladung ist,

sowie mit einem Versorgungssystem für Zusatzgas verbunden sind und jede Elektrode in Gestalt eines Rohres einen zentralen Kanal für die Strömung des Zusatzgases bildet.

**12.** Vorrichtung für die Oberflächenbehandlung durch ein atmosphärisches Plasma nach Anspruch 1, **dadurch gekennzeichnet, dass** sie zumindest zwei Elektroden in Gestalt von Rohren umfasst, deren Achsen sich unter einem Winkel schneiden, der zwischen 0° und 180° variieren kann, und die an einen Stromversorgungskreis angeschlossen sind, der in der Lage ist, eine elektrische Feldintensität E aufrechtzuerhalten, die eine Entladung erzeugt, die der Bedingung gehorcht:

$$(J\,n\,Q\,/\,e)_{Zusatzgas} \leq E \ll (J\,n\,Q\,/\,e)_{Umgebungsgas},$$

WO

J die Aktivierungsenergie der Gasteilchen,

n die Dichte der Teilchen dieses Gases,

Q der Wirkungsquerschnitt der elastischen Zusammenstösse zwischen Elektronen und den Teilchen dieses Gases und

e die Elektronenladung ist.

**13.** Vorrichtung nach Anspruch 12, **dadurch gekennzeichnet, dass** sich der Schnittpunkt der Achsen der rohrförmigen Elektroden unter der zu behandelnden Oberfläche befindet, was die von den Elektroden ausgehenden Plasmaströme zwingt, der zu behandelnden Oberfläche entlang zu verlaufen.

**14.** Vorrichtung nach Anspruch 12, **dadurch gekennzeichnet, dass** einer oder mehrere der Gasströme vermittels zylindrischer oder abgeflachter, zwischen den rohrförmigen Elektroden angeordneter Düsen so auf die zu behandelnde Oberfläche gerichtet werden, dass die Zusammensetzung des aktivierten Gases gegen die zu behandelnde Oberfläche modifiziert wird.

**15.** Vorrichtung nach Anspruch 12, **dadurch gekennzeichnet, dass** eine Mehrzahl von Paaren von rohrförmigen Elektroden so hintereinander angeordnet sind, dass sie einen Kamm bilden, der es erlaubt, eine grosse Oberfläche des zu behandelnden Gegenstandes zu überstreichen.

**16.** Vorrichtung nach einem der vorangehenden Ansprüche, Mittel für die Einstellung der Winkel zwischen den Elektroden und des Abstandes zwischen den Elektroden umfassend.

**17.** Vorrichtung nach einem der vorangehenden Ansprüche, Mittel für die Regelung des Durchsatzes der durch die rohrförmigen Elektroden eingeführten Gase, des Durchsatzes der zwischen den Plasmastrahlen eingeführten Gase, des Stromes und der Spannung zwischen den Elektroden umfassend.

**18.** Vorrichtung nach einem der vorangehenden Ansprüche, Mittel für die Einstellung des Abstandes der Elektroden von der Oberfläche des zu behandelnden Materials umfassend.

**19.** Vorrichtung nach Anspruch 12, **dadurch gekennzeichnet, dass** die rohrförmigen Elektroden so angeordnet sind, dass ihre Achsen parallel sind.

**20.** Vorrichtung nach Anspruch 12, ein System von wabenartig längs angeordneten Rohren umfassend, wobei die Rohre eine variable Länge aufweisen und dafür bestimmt sind, die Geschwindigkeitsverteilung im Zusatzgasstrom auszurichten, um Turbulenzbildung in dem von den Elektroden ausgehenden Plasmastrom zu vermeiden.

**21.** Vorrichtung nach Anspruch 12, eine Düse mit radialen Bohrungen umfassend, die zwischen den Elektroden angeordnet ist, um zu gewährleisten, dass die Verteilung des zwischen die rohrförmigen Elektroden injizierten Gasstromes radial und in Richtung der Plasmastrahlen erfolgt.

22. Vorrichtung nach Anspruch 12, **dadurch gekennzeichnet, dass** sie drei rohrförmige Elektroden umfasst, die von einer Dreiphasenstromquelle versorgt werden, wobei das Zusatzgas vermittels eines Rohres zugeführt wird, das koaxial und abstandsgleich zu diesen Elektroden ist.

23. Vorrichtung nach Anspruch 12, **dadurch gekennzeichnet, dass** ein gekühlter Magnetfeldgenerator zwischen den Elektroden angeordnet ist, der ein Feld senkrecht zu der zu behandelnden Oberfläche erzeugt.

24. Vorrichtung nach Anspruch 11 oder 12, **dadurch gekennzeichnet, dass** die Stromquelle für die Versorgung der elektrischen Entladung eine Gleichstromquelle ist.

25. Vorrichtung nach Anspruch 11 oder 12, **dadurch gekennzeichnet, dass** die Stromquelle für die Versorgung der elektrischen Entladung eine Dreiphasen-Wechselstromquelle ist.

26. Vorrichtung nach Anspruch 11 oder 12, **dadurch gekennzeichnet, dass** die Stromquelle für die Versorgung der elektrischen Entladung eine Quelle von Impulsen ist, deren Vorderflanke, Dauer und Rückenflanke regelbar sind.

27. Vorrichtung nach Anspruch 11 oder 12, **dadurch gekennzeichnet, dass** die rohrförmige(n) Elektrode(n) in Gestalt von Rohren aus dielektrischem Material gefertigt sind, die von koaxialen Metallrohren umgeben sind, von einer Hochfrequenz-Stromquelle versorgt und durch ein auf die Plasmazone gerichtetes Gas gekühlt werden.

28. Vorrichtung nach Anspruch 11 oder 12, **dadurch gekennzeichnet, dass** die rohrförmige(n) Elektrode(n) in Gestalt von konischen Rohren gefertigt sind, um die Ströme des Zusatzgases auf den zu behandelnden Bereich zu lokalisieren.

29. Vorrichtung nach Anspruch 11 oder 12, **dadurch gekennzeichnet, dass** die rohrförmige(n) Elektrode(n) von aussen gekühlt und innen mit rohrförmigen Ringen ausgerüstet sind, um den von den Elektrodenflecken ausgehenden Wärmestrom zu zerstreuen.

30. Vorrichtung nach Anspruch 11 oder 12, **dadurch gekennzeichnet, dass** die rohrförmige(n) Elektrode(n) in Gestalt von Überschall- oder Lavaldüsen gefertigt sind, um die hydrodynamischen Kräfte für das Zusammendrücken des Plasmas auf der zu behandelnden Oberfläche zu vergrössem, indem über der zu behandelnden Oberfläche eine Schockwelle erzeugt wird.

31. Vorrichtung nach Anspruch 11, **dadurch gekennzeichnet, dass** die zweite Elektrode die Gestalt eines hohlen Kegelstumpfes besitzt, der koaxial um die rohrförmige Elektrode herum angeordnet ist.

32. Vorrichtung nach Anspruch 11, **dadurch gekennzeichnet, dass** die zweite Elektrode stabförmig ist.

33. Verwendung einer Vorrichtung nach einem der vorangehenden Ansprüche für die Behandlung textiler Materialien, um ihnen hydrophile oder hydrophobe Eigenschaften zu verleihen.

34. Verwendung einer Vorrichtung nach einem der vorangehenden Ansprüche für die Behandlung keramischer Materialien, um deren hydrophile oder hydrophobe Eigenschaften zu gewährleisten.

35. Verwendung einer Vorrichtung nach einem der vorangehenden Ansprüche zur Kauterisation von Blut und medizinisch-biologischen Geweben bei chirurgischen Eingriffen.

36. Verwendung einer Vorrichtung nach einem der vorangehenden Ansprüche zum Stoppen von Absonderungen von Lymphflüssigkeit bei Verbrennungen.

37. Verwendung einer Vorrichtung nach einem der vorangehenden Ansprüche für die Oberflächenaktivierung von metallischen, schlecht leitenden und dielektrischen Gegenständen, um ihre Adhäsion an Anstrichen, Leim oder anderen Substanzen zu erhöhen.

FIG. 1a

FIG. 1b

FIG. 1c

FIG. 1d

FIG. 2b

FIG. 2a

FIG. 3b

12a, 12b, 12e

$Q_1$   $Q_3$   $Q_1$

6   6
36
32
24
34
4a

α

4b

8b   8a
14b   14a
16
10

37
24

4b   4a

FIG. 3a

FIG. 4

FIG. 5

FIG.7a

←12a

SCHEMA 1

FIG.7b

←12b

SCHEMA 2

FIG.7c

←12c

SCHEMA 3

FIG.7d

←12d

SCHEMA 4

FIG.7e

←12e

SCHEMA 5

FIG. 6

FIG. 8

FIG.9

FIG. 10

**FIG. 11 b**

**FIG. 11 a**

FIG. 12

FIG. 13

FIG. 14

FIG. 15

FIG.16a

FIG.16b

FIG.16c

FIG.16d

FIG. 17a (tableau 1)

PE - polyéthylène
PP - polypropylène
PET - polyéthylènethéréphtolate

*FIG. 17 b* (tableau 2)

*FIG. 17c* (tableau 3)

*FIG. 17 d* (tableau 4)

*FIG. 17 e* (tableau 5)

**FIG. 17 f** (tableau 6)

A.n.- *Aspergillus niger* ATCC 16404

B.n.- *Byssochlamys nivea* 1910-90

*FIG. 17 g* (tableau 8)

*FIG. 17 h* (tableau 8)